# EUROPEAN PATENT SPECIFICATION

(11) **EP 0 806 410 B1**
(45) Date of publication and mention of the grant of the patent: **11.07.2001**
(21) Application number: 97303043.0
(22) Date of filing: 02.05.1997
(51) Int. Cl.: C07C 69/757, C07C 69/753, C07C 251/44, C07C 61/39, C07C 229/08, C07C 271/22, C07C 237/36, A61K 31/215

(54) **Anti-viral compounds**
Antivirusverbindungen
Composés antiviraux

(30) Priority: 06.05.1996 US 16901
(43) Date of publication of application: 12.11.1997
(73) Proprietor: ELI LILLY AND COMPANY, Indianapolis, Indiana 46285 (US)
(72) Inventor: Hornback, William Joseph, Fishers, Indiana 46038 (US); Mauldin, Scott Carl, Indianapolis, Indiana 46221 (US); Munroe, John Edwin, Indianapolis, Indiana 46220 (US)
(74) Representative: Hudson, Christopher Mark

(56) References cited:
- US-A- 2 750 405
- CHEMICAL ABSTRACTS, vol. 50, no. 4, 25 February 1956 Columbus, Ohio, US; abstract no. 2502i, MICHITOSHI OHTA: column 2502; XP002061230 & MISHITOSHI OHTA: "Azaditerpenoids.II. Beckmann rearrangement of methyl 9-hydroxyiminohydroabietate" J.PHARM.SOC.JAPAN, vol. 75, 1955, pages 289-292,
- G.DEFAYE-DUCHATEAU: "Oxydations dans la série de l'acide déhydroabiétique" BULLETIN DE LA SOCIETE CHIMIQUE DE FRANCE., 1964, PARIS FR, pages 1469-1473, XP002061227
- GEORGES DUPONT ET AL.: "Oxydation de l'acide abiétique par l'acétate mercurique.Dérivés de l'acide déhydroabiétique substitués dans le cycle B." BULLETIN DE LA SOCIETE CHIMIQUE DE FRANCE., 1955, PARIS FR, pages 708-715, XP002061228
- J.C.SIRCAR ET AL.: "Free-Radical Bromination of Methyl Abietate by N-Bromosuccinimide and solvolysis of the Products" JOURNAL OF ORGANIC CHEMISTRY., vol. 35, no. 9, September 1970, EASTON US, pages 3090-3093, XP002061229

## Description

Influenza viruses cause an infectious disease for which there is no adequate therapeutic agent. The disadvantages of existing treatments include the onset of clinical resistance within thirty six hours and the ineffectiveness of the agents against influenza B. Killed influenza virus vaccines have been available for over sixty years. However, these vaccines have not lessened the morbidity, mortality or severe financial loss caused by this disease. It follows that an agent which treats or prevents an influenza infection or is effective at preventing the clinical symptoms associated with an influenza infection will result in a significant benefit to society.

Currently, the only compounds approved for the therapeutic and prophylactic treatment of influenza infections are the adamantanes: amantadine and rimantadine. These compounds inhibit influenza A by inhibiting the function of the M2 ion channel activity of the virus. Amantadine is a potent *in vitro* inhibitor of influenza A virus as demonstrated by standard antiviral assays such as the plaque reduction assay. Amantadine is effective in reducing the duration of fever and other systemic complaints including but not limited to myalgia (muscular ache) and fatigue when administered to individuals infected with influenza A within forty-eight hours of the onset of clinical symptoms. It has also been observed that amantadine results in a one hundred-fold decrease of virus titer in the nasal washes of human volunteers infected with wild-type influenza virus which correlates with a dramatic decrease in fever score. Thus, *in vitro* influenza inhibition is predictive of useful *in vivo* effects, i.e. a reduction of the clinical symptoms associated with the influenza infection.

The present invention derives from the fact that influenza is an enveloped virus which dictates that the virus envelope must be fused with the endosomal membrane of the host cell in order to initiate the process of introducing its genetic information into the cell. Because this process is common to all enveloped viruses, it is an attractive target for antiviral chemotherapy. Examples of envelope viruses which are inhibited according to the present invention include influenza, bovine diarrheal, hepatitis C, tick borne encephalitis and the like. The fusion domain of the envelope glycoprotein of influenza, hemagglutinin (HA) has been well-characterized. See, White J.M., Annu. Rev. Physiol. vol. 52, pages 675-697 (1990) which is herein incorporated by reference.

Influenza virus HA provides at least two distinct functions: 1) recognition of the host cell receptor, *i.e.*, sialic acid residues on glycoconjugates, and 2) fusion of the viral envelope with the endosomal membrane. Both functions are essential for the propagation of influenza virus *in vitro* and *in vivo.* During viral maturation, monomeric HA is inserted into a lipid bilayer, post-translationally modified and oligomerized into a trimer of identical subunits (trimeric HA). The infectivity of the progeny virus is contingent upon a site-specific cleavage of HA by host cell protease(s). This cleavage results in the formation of two polypeptide chains, HA1 and HA2, which remain associated by non-covalent interactions as well as by an intermolecular and intramolecular disulfide bonds.

It has been established that influenza HA has two functionally relevant conformations. One conformation (Form A) exists as a metastable structure at neutral pH and mediates receptor recognition. Following receptor mediated binding to the host cell, the virus is transported to the endosomal compartment where it encounters an acidic environment. The low pH triggers a dramatic structural rearrangement of HA (Form A) which results in the formation of the other, more stable conformation of HA (Form B).

Form B of HA is required for fusion of the virus envelope with the endosomal membrane. It is the structural rearrangement from Form A to Form B of HA that allows the fusion domain of HA to directly interact with the endosomal membrane enabling the release of viral genetic information into the host cell cytoplasm. These considerations lend themselves to the development of a strategy for antiviral intervention based on the abrogation of HA-mediated fusion of virus-host membranes.

US 2,750,405, Sircar et al., J. Org. Chem., 35, 1970, pages 3090 to 3093 and DuPont et al., Bulletin de la Societe Chimique de France 1955, pages 708-715 disclose certain derivatives of dehydroabietic acid.

The present invention relates to a compound of the formula: 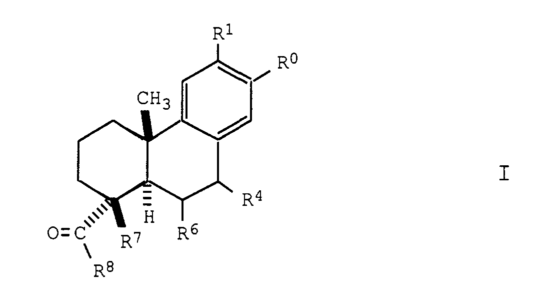 wherein:
R⁰ and R¹ are independently hydrogen, hydroxy, C₁-C₆ alkyl, C₁-C₆ alkoxy, hydroxy(C₁-C₆ alkyl), sulfhydryl, sulfamyl, -SO₂-Cl, -S-C(O)-N(CH₃)₂, amino, C₁-C₄ alkylamino, di(C₁-C₄ alkyl)amino, C₁-C₄ alkylsulfonylamino, di(C₁-C₄ alkylsulfonyl)amino -X⁰-O-C(O)-C₁-C₄ alkyl, -O-(X¹)ᵢ-X², -C(O)-X³, -N-C(O)-R² or -O-R³;
X⁰ is a bond or divalent(C₁-C₆ alkyl);
X¹ is an amino acid;
X² is hydrogen or an amino protecting group;
i is 1, 2 or 3;
X³ is C₁-C₆ alkyl, C₁-C₆ alkoxy, halo(C₁-C₆ alkyl), hydroxy(C₁-C₆ alkyl) or phenyl;
R² is C₁-C₄ alkyl, C₁-C₄ alkoxy, halo(C₁-C₄ alkyl), hydroxy(C₁-C₄ alkyl), phenyl, p-methoxy-phenyl, p-fluorophenyl, naphthyl, pyridyl, piperidinyl, thiazolyl, oxazolyl, thienyl, furyl, tetrahydrofuryl or cyclohexyl;
R³ is C₂-C₆ alkenyl, -CH₂-R^{3a}, -C(O)-R^{3b}, -C(S)-R^{3c}, -C(CH₃)₂C(O)NH₂, phenyl or a group of the formula: 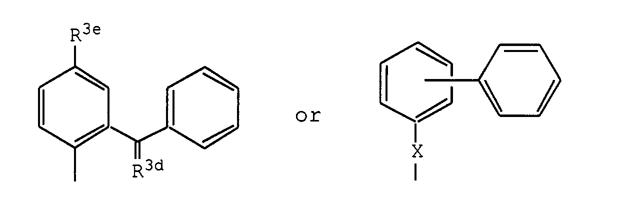
R^{3a} is phenyl, p-fluorophenyl, pyridyl, pyrrolidinyl, piperidinyl, piperazinyl, morpholinyl, N-(C₁-C₄ alkoxycarbonyl)piperidinyl, N- (trifluoromethyl) -piperidinyl, thiazolyl, oxazolyl, imidazolyl, isothiazolyl, isooxazolyl, quinolyl, isoquinolyl, thienyl, furyl, tetrahydrothienyl, tetrahydrofuryl, cyclohexyl, cyclopentyl, cyclopropyl or naphthyl;
R^{3b} is pyrrolidinyl, piperidinyl, piperazinyl, morpholinyl, N-(C₁-C₄ alkoxycarbonyl)piperidinyl, N-(trifluoromethyl)piperidinyl, benzyloxy, pyridylmethyloxy, C₁-C₆ alkoxy, halo(C₁-C₄ alkoxy), amino, C₁-C₄ alkylamino or di(C₁-C₄ alkyl)amino;
R^{3c} is amino, C₁-C₄ alkylamino or di(C₁-C₄ alkyl)amino;
R^{3d} is oxygen, hydroximino, hydrazino or =CHZ;
Z is hydrogen, C₁-C₄ alkyl, halogen, di(C₁-C₄ alkyl)amino, C₁-C₄ alkoxycarbonyl, carbamoyl(C₁-C₄ alkyl), N-(C₁-C₄ alkyl)carbamoyl or N,N-di(C₁-C₄ alkyl)carbamoyl;
R^{3e} is hydrogen, nitro or trifluoromethyl;
X is a bond or -(CH₂)-;
R⁴ is hydrogen, hydroxy, amino, C₁-C₄ alkylamino, di(C₁-C₄ alkyl)amino, C₁-C₄ alkoxy, =O, -O-S(CH₃)₂C(CH₃)₃, C₂-C₆ alkanoyloxy, N-(C₂-C₆ alkanoyl)amino, =N-R⁵ or R⁴ and R⁶ combine to form a bond;
R⁵ is hydroxy, amino, C₁-C₄ alkylamino, di(C₁-C₄ alkyl)amino, C₁-C₄ alkoxy, pyridylmethoxy, benzyloxy, piperazinyl, N-(methyl)piperazinyl or -O-CH₂-C(O) -R^{5a};
R^{5a} is hydroxy or C₁-C₄ alkoxy;
R⁶ is hydrogen, halo, C₁-C₄ alkyl or =O;
R⁷ is hydrogen or C₁-C₄ alkyl;
R⁸ is hydroxy, halo, C₁-C₆ alkoxy, pyrrolidinyl, piperidinyl, piperazinyl, 4-methyl-piperazinyl, morpholinyl or -N(R⁹) -R¹⁰;
R⁹ is hydrogen or methyl;
R¹⁰ is -(divalent C₁-C₆ alkyl)-R^{10a};
R^{10a} is pyridyl,
with the proviso that
i) when R⁴ is hydrogen or =O; R⁸ is methoxy; and R¹ is hydrogen; then R⁰ cannot be hydrogen, hydroxy, methoxy, -C(CH₃)₂OH, -C(O)CH₃, -OC(O)CH₃ or -C (CH₃)₂OC(O)CH₃;
ii) when R⁴ is hydrogen or =O; R⁸ is methoxy; and R⁰ is isopropyl; then R¹ cannot be hydroxy or methoxy;
iii) when R⁴ is hydrogen or =O; R⁸ is hydroxy; and R⁰ is hydrogen; then R¹ cannot be hydrogen or isopropyl;
iv) when R⁴ is hydrogen; R⁸ is methoxy; and R⁰ is hydrogen; then R¹ cannot be hydrogen, hydroxy, -C(O)CH₃ or -OC(O)CH₃;
v) when R¹ is hydrogen; and R⁰ is isopropyl; then R⁴ cannot be hydrogen, hydroxy, methoxy, ethoxy, =O, -OC(O)CH₃, =NOH or combine with R⁶ to form a bond;
vi) when R¹ is hydrogen; R⁴ is hydrogen and R⁸ is hydroxy; then R⁰ cannot be -C (CH₃)₂OC(O)CH₃;
or a pharmaceutically acceptable salt thereof.

The present invention provides new compounds of formula I, as described above, that are useful for treating or preventing a viral infection where the virus is an envelope virus that undergoes hemagglutinin-mediated fusion with a host cell and/or the resultant symptoms. These compounds, their pharmaceutically acceptable salts and the corresponding pharmaceutical formulations can be used alone or in combination with other antivirals, immunomodulators, antibiotics or vaccines.

All temperatures stated herein are in degrees Celsius (°C). All units of measurement employed herein are in weight units except for liquids which are in volume units.

The term "halo" represents chloro, fluoro, bromo or iodo.

The term "C₁-C₆ alkyl" represents a straight or branched alkyl chain having from one to six carbon atoms. Typical C₁-C₆ alkyl groups include methyl, ethyl, propyl, isopropyl, butyl, isobutyl, sec-butyl, t-butyl and the like. The term "C₁-C₆ alkyl" includes within its definition the term "C₁-C₄ alkyl."

The term "halo(C₁-C₆)alkyl" represents a straight or branched alkyl chain having from one to six carbon atoms with 1, 2 or 3 halogen atoms attached to it. Typical halo(C₁-C₆)alkyl groups include chloromethyl, 2-bromoethyl, 1-chloroisopropyl, 3-fluoropropyl, 2,3-dibromobutyl, 3-chloroisobutyl, iodo-t-butyl, trifluoromethyl and the like.

The term "hydroxy(C₁-C₆)alkyl" represents a straight or branched alkyl chain having from one to six carbon atoms with an hydroxy group attached to it. Typical hydroxy(C₁-C₆)alkyl groups include hydroxymethyl, 2-hydroxyethyl, 1-hydroxyisopropyl, 2-hydroxypropyl, 2-hydroxybutyl, 3-hydroxyisobutyl, hydroxy-t-butyl, hydroxypentyl and the like.

The term "C₁-C₄ alkylamino" represents a straight or branched alkylamino chain having from one to four carbon atoms attached to an amino group. Typical C₁-C₄ alkyl-amino groups include methylamino, ethylamino, propylamino, isopropylamino, butylamino, sec-butylamino and the like.

The term "di(C₁-C₄)alkylamino" represents a straight or branched dialkylamino chain having two alkyl chains, each having independently from one to four carbon atoms attached to a common amino group. Typical di(C₁-C₄)alkylamino groups include dimethylamino, ethylmethylamino, methylisopropylamino, t-butylisopropylamino, di-t-butylamino and the like.

The term "C₁-C₆ alkoxy" represents a straight or branched alkyl chain having from one to six carbon atoms attached to an oxygen atom. Typical C₁-C₆ alkoxy groups include methoxy, ethoxy, propoxy, isopropoxy, butoxy, t-butoxy, pentoxy and the like. The term "C₁-C₆ alkoxy" includes within its definition the term "C₁-C₄ alkoxy".

The term "C₂-C₆ alkenyl" represents a straight or branched alkenyl chain having from two to six carbon atoms. Typical C₂-C₆ alkenyl groups include ethenyl, propenyl, isopropenyl, buten-2-yl, t-butenyl, penten-1-yl, hexen-3-yl, 3-methylpentenyl and the like.

The term "C₁-C₄ alkoxycarbonyl" represents a straight or branched alkoxy chain having from one to four carbon atoms attached to a carbonyl moiety. Typical C₁-C₄ alkoxycarbonyl groups include methoxycarbonyl, ethoxycarbonyl, propoxycarbonyl, isopropoxycarbonyl, butoxycarbonyl, t-butoxycarbonyl and the like.

The term "carbamoyl (C₁-C₄) alkyl" represents a straight or branched alkyl chain having from one to four carbon atoms with a carbamoyl group attached to it. Typical carbamoyl (C₁-C₄) alkyl groups include carbamoylmethyl, carbamoylethyl, carbamoylpropyl, carbamoylisopropyl, carbamoylbutyl and carbamoyl-t-butyl and the like.

The term "N-(C₁-C₄)alkylcarbamoyl" represents a straight or branched alkyl chain having from one to four carbon atoms attached to the nitrogen atom of a carbamoyl moiety. Typical N-(C₁-C₄ alkyl)carbamoyl groups include N-methylcarbamoyl, N-ethylcarbamoyl, N-propylcarbamoyl, N-isopropylcarbamoyl, N-butylcarbamoyl, N-t-butylcarbamoyl and the like.

The term "N,N-di(C₁-C₄ alkyl) carbamoyl" represents a straight or branched alkyl chain having a straight or branched C₁-C₄ alkyl chain attached to each of the nitrogen atoms on a carbamoyl moiety. Typical N-(C₁-C₄)alkylcarbamoyl groups include N,N-dimethylcarbamoyl, N-ethyl-N-methylcarbamoyl, N-propyl-N-butylcarbamoyl, N,N-diisopropylcarbamoyl, N-methyl-N-butylcarbamoyl and the like.

The term "C₁-C₄ alkylsulfonylamino" represents a straight or branched alkyl group having from one to four carbon atoms attached to a sulfonylamino moiety. TypicalC₁-C₄ alkylsulfonylamino groups include methylsulfonyl-amino, ethylsulfonylamino, propylsulfonylamino, isopropylsulfonylamino, butylsulfonylamino, isobutylsulfonylamino, sec-butylsulfonylamino and t-butylsulfonylamino.

The term "di(C₁-C₄ alkylsulfonyl) amino" represents two C₁-C₄ alkylsulfonyl moieties attached to an amino moiety. Typical di(C₁-C₄ alkylsulfonyl) amino groups include methylmethylsulfonylamino, ethylmethylsulfonylamino, propylethylsulfonylamino, isopropylmethylsulfonylamino, t-butylethylsulfonylamino, butylbutylsulfonylamino and the like

The term "C₂-C₆ alkanoyl" represents a straight or branched alkyl chain having from one to five carbon atoms attached to a carbonyl moiety. Typical C₂-C₆ alkanoyl groups include ethanoyl, propanoyl, isopropanoyl, butanoyl, *t*-butanoyl, pentanoyl, hexanoyl, 3-methylpentanoyl and the like.

The term "C₂-C₆ alkanoyloxy" represents a straight or branched alkyl group having from one to five carbon atoms attached to a carbonyloxy moiety. Typical C₂-C₆ alkanoyloxy groups include ethanoyloxy, propanoyloxy, isopropanoyloxy, butanoyloxy, isobutanoyloxy, sec-butanoyloxy, t-butanoyloxy, pentanoyloxy and the like.

The term "C₂-C₆ alkanoylamino" represents a straight or branched alkyl group one to five carbon atoms attached to a carbonylamino moiety. Typical C₂-C₆ alkanoylamino groups include ethanoylamino, propanoylamino, isopropanoylamino, butanoyl-amino, isobutanoylamino, sec-butanoylamino, t-butanoylamino, pentanoylamino and the like.

As mentioned above, the invention includes the pharmaceutically acceptable salts of the compounds defined by formula I. Although generally neutral, a compound of this invention can possess a sufficiently acidic, a sufficiently basic, or both functional groups, and accordingly react with any of a number of inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a mineral or organic acid or an inorganic base. Such salts are known as acid addition and base addition salts.

Acids commonly employed to form acid addition salts are inorganic acids such as hydrochloric acid, hydrobromic acid, hydroiodic acid, sulfuric acid, phosphoric acid and the like, and organic acids such as *p*-toluenesulfonic, methanesulfonic acid, oxalic acid, *p*-bromophenylsulfonic acid, carbonic acid, succinic acid, citric acid, benzoic acid, acetic acid, and the like.

Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, γ-hydroxybutyrate, glycollate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counterion forming a part of any salt of this invention is not of a critical nature, so long as the salt as a whole is pharmacologically acceptable and as long as the counterion does not contribute undesired qualities to the salt as a whole.

The term "amino-protecting group" as used in the specification refers to substituents of the amino group commonly employed to block or protect the amino functionality while reacting other functional groups on the compound. Examples of such amino-protecting groups include formyl, trityl, phthalimido, trichloroacetyl, chloroacetyl, bromoacetyl, iodoacetyl groups, or urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, *t*-butoxycarbonyl, 2-(4-xenyl)isopropoxycarbonyl, 1,1-diphenyleth-1-yloxycarbonyl, 1,1-diphenylprop-l-yloxycarbonyl, 2-phenylprop-2-yloxycarbonyl, 2-(*p*-toluyl)-prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)-ethoxycarbonyl, 2-(methylsulfonyl)ethoxycarbonyl, 2-(triphenylphosphino)-ethoxycarbonyl, fluorenylmethoxycarbonyl ("FMOC"), 2-(trimethylsilyl)ethoxycarbonyl, allyloxycarbonyl, 1- (trimethylsilylmethyl)prop-l-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; benzoylmethylsulfonyl, 2-nitrophenylsulfenyl, diphenylphosphine oxide and like amino-protecting groups. The species of amino-protecting group employed is not critical so long as the derivatized amino group is stable to the condition of subsequent reaction(s) on other positions of the intermediate molecule and can be selectively removed at the appropriate point without disrupting the remainder of the molecule including any other amino-protecting group(s). Preferred amino-protecting groups are t-butoxycarbonyl (t-Boc), allyloxycarbonyl and benzyloxycarbonyl (CbZ). Further examples of groups referred to by the above terms are described by J. W. Barton, "Protective Groups in Organic Chemistry", J. G. W. McOmie, Ed., Plenum Press, New York, N.Y., 1973, Chapter 2, and T. W. Greene, "Protective Groups in Organic Synthesis", John Wiley and sons, New York, N.Y., 1981, Chapter 7.

The term "carboxy-protecting group" as used in the specification refers to substituents of the carboxy group commonly employed to block or protect the carboxy functionality while reacting other functional groups on the compound. Examples of such carboxy-protecting groups include methyl, p-nitrobenzyl, p-methylbenzyl, p-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl, 4-methoxytrityl, 4,4'-dimethoxytrityl, 4,4',4''-trimethoxytrityl, 2-phenylprop-2-yl, trimethylsilyl, t-butyldimethylsilyl, phenacyl, 2,2,2-trichloroethyl, β-(dibutylmethylsilyl)ethyl, p-toluenesulfonylethyl, 4-nitrobenzylsulfonylethyl, allyl, cinnamyl, 1- (trimethylsilylmethyl)prop-1-en-3-yl and like moieties. Preferred carboxy-protecting groups are allyl, benzyl and t-butyl. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5.

Preferred compounds are those compounds of formula I where:
R⁰ is hydrogen, hydroxy, C₁-C₆ alkyl, C₁-C₆ alkoxy, hydroxy(C₁-C₆ alkyl), -X⁰-O-C(O)-C₁-C₄ alkyl, -O-(X¹)ᵢ-X², -C(O)-X³ or -O-R³;
R¹ is hydrogen, hydroxy, C₁-C₆ alkoxy, sulfhydryl, sulfamyl, -SO₂-Cl, amino, di(C₁-C₄ alkylsulfonyl)amino, -C(O)-X³, -N-C(O)-R² or -O-R³;
X⁰ is a bond or divalent(C₁-C₆ alkyl);
X¹ is an amino acid;
X² is hydrogen or an amino protecting group;
i is 1 or 2;
X³ is C₁-C₆ alkyl;
R² is hydroxy(C₁-C₄ alkyl);
R³ is C₂-C₆ alkenyl, -CH₂-R^{3a}, -C(O)-R^{3b}, -C(S)-R^{3c}, -C(CH₃)₂C(O)NH₂ or a group of the formula: 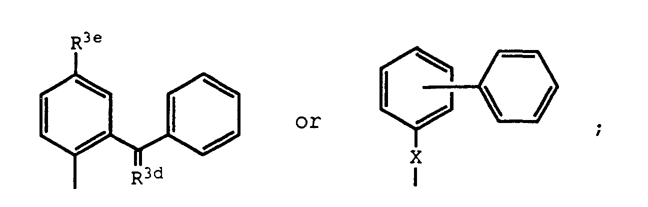
R^{3a} is phenyl, p-fluorophenyl, pyridyl, piperidinyl, piperazinyl or morpholinyl;
R^{3b} is piperidinyl, piperazinyl, morpholinyl, N-(C₁-C₄ alkoxycarbonyl)piperidinyl, N-(trifluoromethyl)piperidinyl, halo(C₁-C₄ alkoxy) or di(C₁-C₄ alkyl)amino;
R^{3c} is di(C₁-C₄ alkyl)amino;
R^{3d} is oxygen or hydroximino;
R^{3e} is hydrogen, nitro or trifluoromethyl;
X is a bond;
R⁴ is hydrogen, hydroxy, amino, =O, C₂-C₆ alkanoyloxy, =N-R⁵ or -OSi(CH₃)₂;
R⁵ is hydroxy, amino, di(C₁-C₄ alkyl)amino, C₁-C₄ alkoxy, pyridylmethoxy, N-(methyl)piperazinyl or -O-CH₂-C(O) -R^{5a};
R⁶ is hydrogen, chloro, bromo, methyl or =O;
R⁷ is hydrogen or methyl;
R⁸ is hydroxy, chloro, methoxy, 4-methylpiperazinyl or -N(R⁹) -R¹⁰;
R⁹ is hydrogen;
R¹⁰ is -CH₂-R^{10a}; and
R^{10a} is pyridyl;
or a pharmaceutically acceptable salt thereof.

Of these compounds, more preferred are those compounds of formula I where:
R⁰ is hydrogen, hydroxy, C₁-C₆ alkoxy, -O-(X¹)ᵢ-X², -X⁰-O-C(O)-C₁-C₄ alkyl or -O-R³;
R¹ is hydrogen, hydroxy, C₁-C₆ alkoxy or -O-R³;
X⁰ is a bond;
X¹ is an amino acid;
X² is hydrogen or an amino protecting group;
i is 1 or 2;
R³ is C₂-C₆ alkenyl, -CH₂-R^{3a} or -C(O)-R^{3b};
R^{3a} is p-fluorophenyl or pyridyl;
R^{3b} is piperidinyl;
R⁴ is hydrogen, hydroxy, =O or =N-R⁵;
R⁵ is hydroxy, dimethylamino or N-(methyl)piperazinyl;
R⁶ is hydrogen, bromo or =O;
R⁷ is methyl; and
R⁸ is methoxy;
or a pharmaceutically acceptable salt thereof.

Of these compounds, even more preferred are those compounds of formula I where:
R⁰ is hydrogen, hydroxy, C₁-C₄ alkoxy, -O-(X¹)ᵢ-X², -O-C(O)methyl or -O-R³;
R¹ is hydrogen, hydroxy, C₁-C₄ alkoxy or -O-R³;
X¹ is glycine, alanine or valine;
X² is hydrogen, t-butoxycarbonyl or benzyloxycarbonyl;
R⁴ is =O or =N-R⁵;
R⁵ is hydroxy;
R⁶ is hydrogen;
or a pharmaceutically acceptable salt thereof.

The compounds of formula I may be prepared according to procedures known in the art. For example, the following Reaction Schemes may be used, alone or in combination to provide the desired compounds. Once a reaction is complete, the intermediate compound may be isolated by procedures well-known in the art, for example, the compound may be crystallized and then collected by filtration, or the reaction solvent may be removed by extraction, evaporation or decantation. The intermediate compound may be further purified, if desired, by common techniques such as crystallization or chromatography over solid supports such as silica gel or alumina, before carrying out the next step of the reaction scheme.

The compounds of formula I where R⁴ is =O or =N-R may be prepared according to the procedures shown below in Reaction Scheme I. 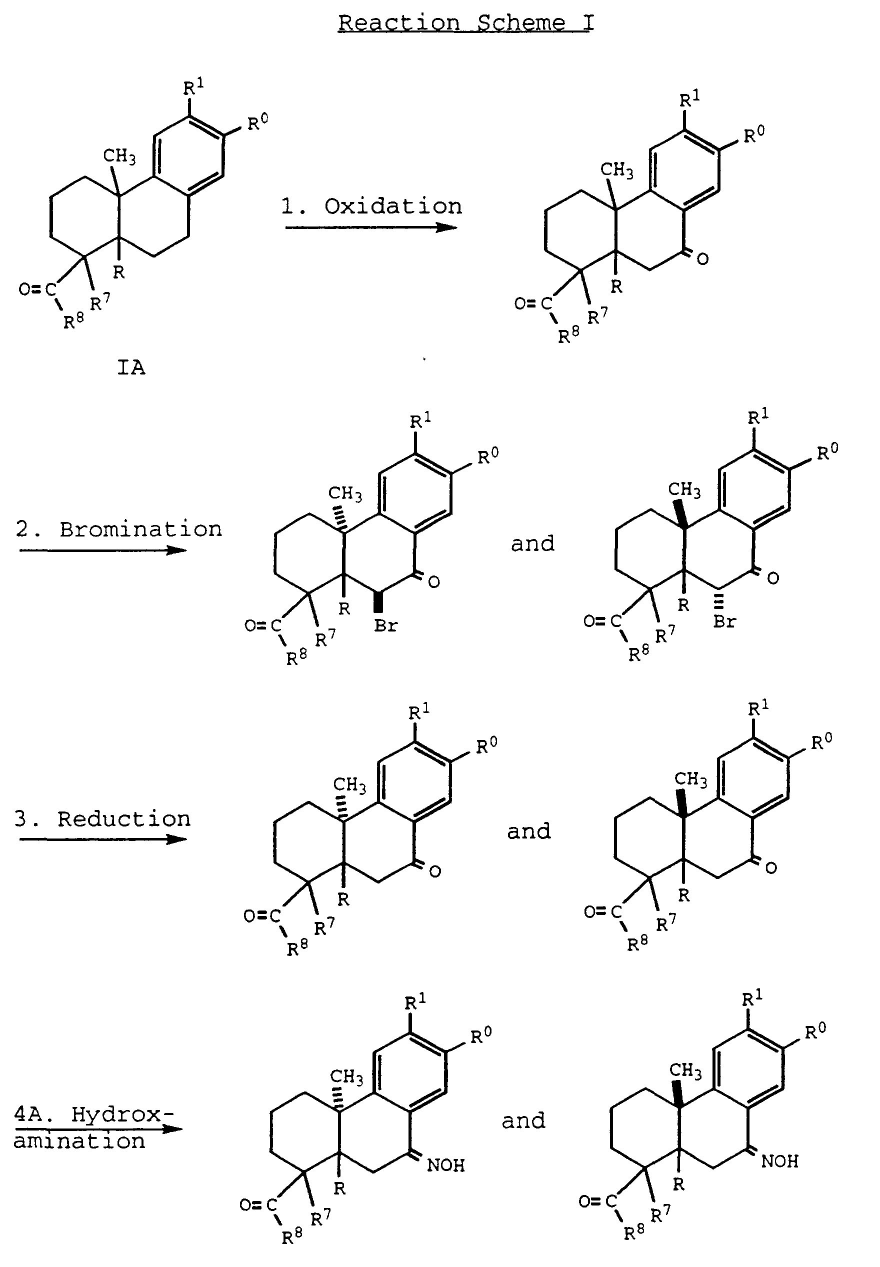 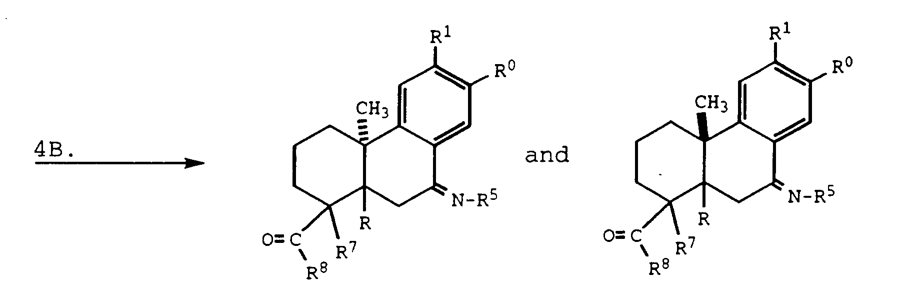 where Reactions I.4A and 4B represent alternative reactions that follow Reaction I.3.

Reaction scheme I is accomplished by carrying out reactions 1-4 is sequential order. Reaction I.1 is carried out by oxidizing a compound of formula IA, for example, by reaction with chromium trioxide in an acetic acid/water mixture, to provide the corresponding ketone. The chromium trioxide is generally employed in an amount ranging from equimolar proportions to about a 4 molar excess relative to the compound of formula IA, preferably in about a 2-4 molar excess. The acetic acid/water mixture is generally a 10:1 to a 2:1 mixture of acetic acid to water, preferably about 4:1. The reaction is generally substantially complete after about 1 to 10 hours when conducted at a temperature of from about 23°C to about 60°C. The reaction is preferably conducted at a temperature of from about 23°C to about 30°C for about 5 to 10 hours.

In Reaction I.2, the ketone obtained from Reaction I.1 is reacted with bromine in a suitable solvent such as diethyl ether, tetrahydrofuran or dimethoxyethane, to provide a mixture of bromoketones which are then separated using standard separation techniques such as chromatography. These isomerically pure bromoketones are then used to prepare various isomerically pure compounds of formula I. The bromine is generally employed in an amount ranging from about equimolar proportions to about a 2 molar excess relative to the ketone reactant, preferably in about a 1-1.5 molar excess. Solvent choice is not critical so long as the solvent employed is inert to the ongoing reaction and the reactants are sufficiently solubilized to effect the desired reaction. The reaction is generally substantially complete after about 1 to 3 hours when conducted at a temperature of from about 23°C to about 30°C. The reaction is preferably conducted at room temperature for about 1 to 1.5 hours.

Alternatively, the ketone obtained from Reaction I.1 is reacted with a silylating agent in the presence of a base in a suitable solvent such as methylene chloride, diethyl ether or tetrahydrofuran to provide the corresponding silylated enol ether. Preferred bases include 2,6-lutidine and collidine. A preferred silylating agent is t-butyldimethylsilyl trifluoromethanesulfonate. The silylating agent is generally employed in an amount ranging from about equimolar proportions to about a 2 molar excess relative to the ketone reactant, preferably in about a 1-1.5 molar excess. Solvent choice is not critical so long as the solvent employed is inert to the ongoing reaction and the reactants are sufficiently solubilized to effect the desired reaction. The reaction is generally substantially complete after about 30 minutes to 2 hours when conducted at a temperature of from about 0°C to about 50°C. The reaction is preferably conducted at a temperature of from about 10°C to about 25°C for about 30 minutes to about 1 hour.

The silylated enol ether is then reacted with bromine substantially as described above with the exception that the reaction is carried out in the presence of acetic acid. Typical solvents suitable for use in this reaction include any organic solvent such as methylene chloride, diethyl ether or tetrahydrofuran. Solvent choice is not critical so long as the solvent employed is inert to the ongoing reaction and the reactants are sufficiently solubilized to effect the desired reaction.

In Reaction I.3, the bromoketone is reduced, for example by reaction with zinc dust and sodium acetate in glacial acetic acid, to provide the corresponding ketones. The zinc is generally employed in an amount ranging from about equimolar proportions to about a 4 molar excess relative to the ketone reactant, preferably in about a 1.5-3 molar excess. The sodium acetate is generally employed in an amount ranging from about 0.6 molar equivalents to about 1.2 molar equivalents relative to the ketone reactant. The reaction is generally substantially complete after about 1 to 10 hours when conducted at a temperature of from about 60°C to the reflux temperature of the mixture. The reaction is preferably conducted at reflux temperature for about 1 to 2 hours.

Alternatively, hydroxylamine hydrochloride is reacted with sodium acetate in a suitable solvent such as ethanol. The sodium acetate is generally employed in an amount ranging from about 1.1 molar equivalents to about a 50 molar excess relative to the hydroxylamine. The reaction is generally substantially complete after about 1 to 72 hours when conducted at a temperature of from about 25°C to about 80°C. The reaction is preferably conducted at a temperature in the range of from about 25°C to about 30°C for about 5 to 24 hours.

In Reaction I.4A, the ketone obtained from Reaction I.3 is reacted with hydroxylamine hydrochloride in a mixture of methanol, water and acetic acid to provide the desired oxime compound. The hydroxylamine hydrochloride is generally employed in an amount ranging from about equimolar proportions to about a 4 molar excess relative to the ketone reactant, preferably in about a 1.3-3 molar excess. The ratio of methanol to water to acetic acid is generally 10-20:1:0.1, preferably 15:1:0.1. The reaction is generally substantially complete after about 1 hour to about 2 days when conducted at a temperature of from about 40°C to the reflux temperature of the mixture. The reaction is preferably conducted at reflux temperature for about 1 to 6 hours.

In Reaction I.4B, the ketone obtained from Reaction I.3 is reacted with an hydrazine hydrochloride such as 1-amino-4-methylpiperazine, 1,1-dimethylhydrazine or hydrazine in the presence of a base in an inert solvent at a temperature of from about 25°C to 80°C for 2 to 24 hours. Typical bases include sodium acetate, potassium hydroxide, triethylamine and the like. Suitable solvents include ethanol, isopropanol and dimethylformamide. Solvent choice is not critical so long as the solvent employed is inert to the ongoing reaction and the reactants are sufficiently solubilized to effect the desired reaction.

The phenyl moiety of the compounds of formula I prepared above may be substituted according to Reaction Scheme II, as follows. 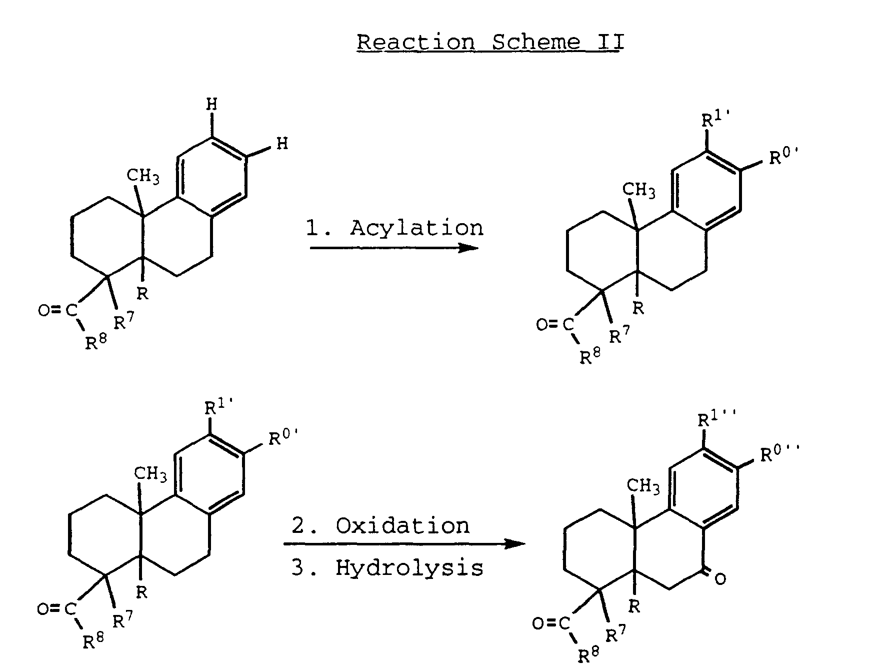 where R⁰' and R¹' are independently hydrogen or -C(O)CH₃; and R⁰'' and R¹" are independently hydrogen or hydroxy.

In Reaction II.1, the compound of formula I where R⁰ and R¹ are each hydrogen is subjected to a Friedel-Crafts acylation by reacting the compound of formula I with an acid halide, in the presence of a catalyst in an inert solvent such as carbon disulfide. The acid halide is generally employed in an amount ranging from about equimolar proportions to about a 1.5 molar excess relative to the compound of formula I, preferably in about a 1.1-1.3 molar excess. Preferred acid halides include acetyl chloride, acetyl bromide or the like. Preferred catalysts include aluminum trichloride, aluminum tribromide or the like. Solvent choice is not critical so long as the solvent employed is inert to the ongoing reaction and the reactants are sufficiently solubilized to effect the desired reaction. The reaction is generally substantially complete after about 1 to 10 hours when conducted at a temperature of from about 50°C to the reflux temperature of the mixture. The reaction is preferably conducted at reflux temperature for about 1 to 2 hours.

In Reaction II.2, the acylated compound of formula I obtained from Reaction II.1 is oxidized to provide the corresponding phenol in a two step reaction. First, the acyl moiety is reacted with a peracid in the presence of an acid catalyst in an inert solvent such as dimethoxyethane to provide the corresponding ester with is then reacted with sodium bicarbonate in an alcohol/water mixture to provide the desired phenol.

The peracid is generally employed in an amount ranging from about equimolar proportions to about a 2 molar excess relative to the acyl moiety, preferably in about a 1-1.3 molar excess. The amount of catalyst typically employed is in the range of 0.005-0.04 equivalents relative to the acyl moiety. A preferred peracid is metachloro-peroxybenzoic acid. A preferred catalyst is p-toluenesulfonic acid. Solvent choice is not critical so long as the solvent employed is inert to the ongoing reaction and the reactants are sufficiently solubilized to effect the desired reaction. The reaction is generally substantially complete after about 1 to 10 hours when conducted at a temperature of from about 50°C to the reflux temperature of the mixture. The reaction is preferably conducted at reflux temperature for about 1 to 3 hours.

The resultant ester is typically refluxed with a base in a methanol/water mixture for about 1 to 7 hours to provide the desired phenol compound. Preferred bases include sodium bicarbonate, sodium carbonate, sodium hydroxide or potassium hydroxide or the like. The base is generally employed in an excess, for example from about a 1 molar excess to about a 6 molar excess relative to the ester moiety, preferably in about a 2-5 molar excess.

The phenol compounds obtained from Reaction Scheme II may be used to prepare various substituted compounds of formula I, as described below.

For example, the hydroxy moiety may be alkylated by reacting the phenol compound with a suitable alkylating agent in the presence of a base in an inert solvent. Examples of bases include triethylamine, diisopropyl ethylamine, sodium hydride and potassium carbonate. Typical solvents include methylene chloride, tetrahydrofuran, dimethylformamide and the like. Solvent choice is not critical so long as the solvent employed is inert to the ongoing reaction and the reactants are sufficiently solubilized to effect the desired reaction. Suitable alkylating agents include iodomethane, allyl iodide, p-fluorophenyl bromide, 3-bromomethyl-pyridine and 2-fluorobenzophenone and the like. The reaction is generally substantially complete after about 1 to 20 hours when conducted at a temperature of from about 0°C to 170°C. The reaction is preferably conducted at a temperature of from about 25°C to about 80°C for about 4 to 16 hours.

Alternatively, the hydroxy moiety may be alkylated by reacting the phenol with an alcohol in the presence of triphenylphosphine and a suitable activating agent in an inert solvent, such as tetrahydrofuran or ethylene glycol dimethyl ether. Examples of suitable activating agents include diethyl azodicarboxylate, dimethyl azodicarboxylate, diisopropyl azodicarboxylate and the like. Examples of alcohols include 3-pyridyl carbinol, N-t-butoxycarbonyl-3-piperidinemethanol and the like. The reaction is generally substantially complete after about 0.5 to 2 hours when conducted at a temperature of from about 0°C to 85°C. The reaction is preferably conducted at a temperature of from about 25°C to about 70°C for about 30 minutes to 1 hour.

The hydroxy moiety may be converted to an ester or a carbonate by reacting the phenol with an acylating agent in the presence of a base in an inert solvent, such as methylene chloride, tetrahydrofuran or dimethylformamide. Typical bases include triethylamine, diisopropyl ethylamine, sodium hydride and the like. Typical acylating agents include N-(t-butoxycarbonyl)-4-chlorocarbonyl piperdine, 2,2,2-trichloroethyl chloroformate, N-(t-butoxycarbonyl)-hydroxybenzotriazole amino esters. The reaction is generally substantially complete after about 1 to 20 hours when conducted at a temperature of from about 0°C to 60°C. The reaction is preferably conducted at a temperature of from about 10°C to about 25°C for about 1 to 5 hours.

The hydroxy moiety may be converted to the corresponding aniline in a three step reaction. First, the phenol is reacted with a suitably substituted amide such as 2-methyl-2-bromo-propanamide in the presence of a base such as sodium hydride or triethylamine in an inert solvent, such as dioxane or tetrahydrofuran at a temperature of 25°C to 100°C to provide the corresponding amido-ether. This amido-ether is then reacted with sodium hydride in an inert solvent such as dimethylformamide, 1,3-dimethyl-3,4,5,6-tetrahydro-2(1H)-pyrimidone or a mixture thereof at temperatures ranging from 25°C to 145°C to provide the rearranged amido-alcohol. Finally, the amido-alcohol is reacted with an acid, such as hydrochloric acid in dioxane at 50°C to 100°C to provide the desired aniline.

The aniline may be converted to the corresponding sulfonamide by reacting the aniline with a sulfonyl chloride such as methanesulfonyl chloride or isopropylsulfonyl chloride in the presence of a base, such as triethylamine, diisopropyl ethylamine or sodium hydride at a temperature of from about 0°C to 50°C in an inert solvent, such as methylene chloride, tetrahydrofuran or dimethylformamide.

The hydroxy moiety may be converted to a thiophenol in a three step reaction. First the phenol is reacted with a thio-carbamoyl (for example dimethylthiocarbamoyl chloride) in the presence of a base in an suitable solvent, such as water or dimethylformamide at a temperature ranging from 25°C to 50°C for 1 to 3 hours to provide the oxo-thiocarbamate. Typical bases include potassium hydroxide, triethylamine and the like. The oxo-thiocarbamate is converted to the corresponding thio-oxocarbamate compound by isolating and heating the neat solid to its melting point. Finally, the thio-oxocarbamate is reacted with a base, such as potassium hydroxide or sodium hydroxide in an alcoholic solvent, such as methanol or ethanol at a temperature of 20°C to 80°C for 20 minutes to 1 hour to provide the corresponding thiophenol.

The thiophenol may be converted to the corresponding sulfonamides by reacting the thiophenol with an oxidizing agent (for example, potassium nitrate) in an inert solvent such as acetonitrile, followed by the addition of a chlorinating agent (for example, sulfuryl chloride) at temperatures ranging from 0°C to 25°C to provide a mixture of sulfonyl chlorides which are separable using standard chromatographic techniques. These sulfonyl chlorides may be converted to the desired sulfonamides by reaction with an appropriately substituted amine such as ammonium hydroxide, methylamine, isopropylamine or benzylamine at a temperature of from about 0°C to 40°C in an inert solvent such tetrahydrofuran.

The hydroxy moiety may be converted to the corresponding amino esters by reacting the phenol with an amino protected amino acid in the presence of a coupling reagent and a catalyst in an inert solvent such as diethyl ether, tetrahydrofuran or methylene chloride. Preferred amino protecting groups include t-butoxycarbonyl or benzyloxycarbonyl. The amino reactant is generally employed in equimolar proportions to a slight excess (1.3 equivalents) relative to the phenol reactant in the presence of an equimolar quantity to a slight excess (1.5 equivalents) of the coupling reagent. Typical coupling agents include dicyclohexylcarbodiimide (DCC), 1-(3-dimethylaminopropyl)-3-ethylcarbodiimide, benzotriazol-1-yloxy-tris(dimethylamino)phosphonium hexafluorophosphate (BOP), N,N'-diethylcarbodiimide, carbonyldiimidazole, bis(2-oxo-3-oxazolidinyl)phosphinic chloride (BOP-Cl) or N-ethoxycarbonyl-2-ethoxy-1,2-dihydroquinoline (EEDQ) and the like. Preferred coupling agents include DCC and BOP. Typical catalysts include DMAP and 4-pyrrolopyridine. The reaction is substantially complete in 1 to 10 hours when carried out at a temperature of from about -30°C to about 35°C, preferably from about 0°C to about 25°C.

The starting materials used in the procedures detailed above may be obtained commercially or prepared according to procedures known in the art. For example, the compounds of formula IA: 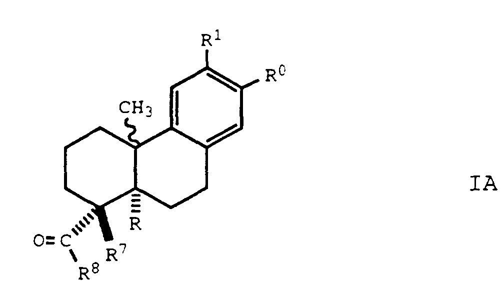 may be prepared substantially in accordance with the procedure detailed in Ohta and Ohmuri, Chem. Pharm. Bull. (Tokyo), vol 5, page 91 (1957). The isomeric mix of compounds may be separated using standard separation techniques such as recrystallization or column chromatography or may be subjected to the bromination methodology described above in Reaction Scheme I. Preferably, these isomers are obtained using the bromination methodology described above in Reaction Scheme I.

The following Preparations and Examples further illustrate specific aspects of the present invention. It is to be understood, however, that these examples are included for illustrative purposes only and are not intended to limit the scope of the invention in any respect and should not be so construed.

### Example 1 (Intermediate)

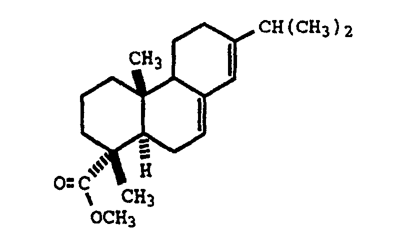

To a solution of NaOMe (prepared in situ from 2.6 g of sodium in 400 ml of anhydrous MeOH (0.108 mol), under N₂), was added 15.0 g (0.035 mol) of 70% abietic acid. After stirring the mixture for 10 minutes, 14.0 ml (0.22 mol) of iodomethane was added and the mixture was refluxed for 24 hours, cooled and concentrated in vacuo to provide a residue which was dissolved in 500 ml of EtOAc, washed sequentially with 500 ml of a saturated NaHCO₃ solution and a saturated sodium chloride solution (NaCl), dried over Na₂SO₄, filtered and concentrated in vacuo. The crude material was purified using flash chromatography (eluent of 2% EtOAc in hexanes). Yield: 10.0 g of a dark yellow oil (90.4%).
IR(CHCl₃): 2952, 1718 and 1251 cm⁻¹.
¹H NMR (300MHz, CDCl₃): δ5.78 (s, 1H); 5.38 (brs, 1H); 3.66 (s, 3H); 2.17-2.30 (m, 3H); 1.68-2.16 (m, 8H); 1.50-1.65 (m, 2H); 1.26 (s, 3H); 1.24 (m, 2H); 1.02 (d, J=2.6Hz, 3H); 1.00 (d, J=2.6Hz, 3H) and 0.83 (s, 3H).
MS(FD): m/e 316(M+).

| Elemental Analysis for C₂₁H₃₂O₂: | | | | |
|---|---|---|---|---|
| Calcd | C, | 79.70; | H, | 10.19; |
| Found | C, | 79.49; | H, | 9.94. |

### Example 2 (Reference compound)

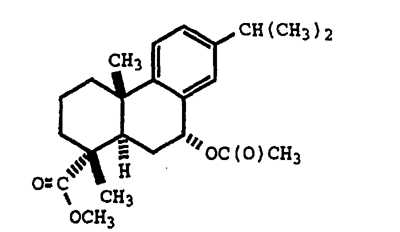

To a mixture of 5.0 g (15.8 mmol) of the compound in Example 1 in 100 ml of acetic anhydride, was added 2.5 g (22.5 mmol) of selenium (IV) oxide, under N₂. The reaction mixture was warmed to 70°C, stirred for 16 hours, cooled, filtered and then diluted to 500 ml with CH₂Cl₂. The resulting layers were separated and the organic layer was washed with 500 ml of NaCl, dried over Na₂SO₄, filtered and then concentrated in vacuo to provide a dark yellow solid. This solid was purified using flash chromatography (eluent of 5% EtOAc in hexanes) to provide two major fractions.

The first fraction was concentrated to provide 537 mg of an oil. This oil was hydrogenated with 135 mg of 5% Pd/C in 25 ml of MeOH (8 hours, room temperature, 6.0 psi). The reaction mixture was filtered and the filtrate concentrated in vacuo. The crude material was purified using flash chromatography (eluent of 2% EtOAc in hexanes) to provide the compound of Example 3 (400 mg of a clear oil (75%) m.p. 50°C). The second fraction was concentrated in vacuo to provide the compound.
Yield: 2.8 g of a light yellow solid (47%).
m.p. 165-167°C.
IR (KBr): 2956, 1722 and 1251 cm⁻¹.
¹H NMR (300MHz, CDCl₃): δ7.23 (m, 2H); 7.04 (d, J=1.8Hz, 1H); 5.90 (m, 1H); 3.64 (s, 3H); 2.86 (m, 1H); 2.60 (dd, J=1.5,11.0Hz, 1H); 2.31 (d, J=12.1Hz, 1H); 2.08 (s, 3H); 2.07 (m, 1H); 1.60-1.80 (m, 6H); 1.26 (s, 3H); 1.24 (s, 3H) ; 1.22 (s, 3H) and 1.19 (s, 3H).
MS(FD): m/e 372(M+).

| Elemental Analysis for C₂₃H₃₂O₄: | | |
|---|---|---|
| Calcd | C, 74.16; | H, 8.66; |
| Found | C, 74.44; | H, 8.71. |

### Example 3 (Reference compound)

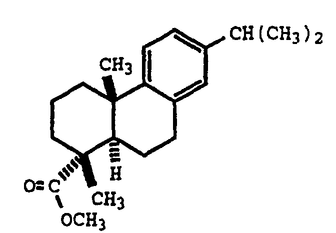

To a mixture of 23.6 g (0.063 mmol) of the compound in Example 2 in 1500 ml of MeOH, was added 5.8 g of 10% Pd/C and 5.8 g (0.030 mmol) of p-toluenesulfonic acid monohydrate. The reaction mixture was reacted for 16 hours at room temperature, 60 psi, filtered and then concentrated in vacuo to provide a residue. This residue was dissolved in 700 ml of EtOAc, washed sequentially with 700 ml of saturated NaHCO₃ and NaCl solutions, dried over Na₂SO₄, filtered and then concentrated in vacuo.
Yield: 19.3 g (97.5%) of an oil.
IR (CHCl₃): 2955, 1718 and 1254 cm⁻¹.
¹H NMR (300MHz, CDCl₃): δ7.16 (d, J=8Hz, 1H); 7.00 (d, J=8Hz, 1H); 6.88 (s, 1H); 3.66 (s, 3H); 2.80-2.90 (m, 3H); 2.23-2.32 (m, 2H); 1.35-1.90 (m, 7H); 1.28 (s, 3H); 1.24 (s, 3H) and 1.21 (s, 6H).
MS(FD): m/e 314(M+).

| Elemental Analysis for C₂₁H₃₀O₂: | | |
|---|---|---|
| Calcd | C, 80.21; | H, 9.62 |
| Found | C, 80.34; | H, 9.73 |

### Example 4 (Reference compound)

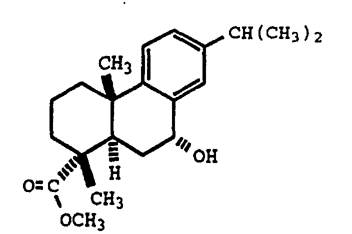

To a suspension of 185 mg (0.50 mmol) of the compound of Example 2 in 10 ml of MeOH, was added 5 ml (0.50 mmol) of 0.1N NaOH. The reaction mixture was refluxed for 2 hours, cooled and partitioned between 50 ml of EtOAc and 50 ml of 0.2N HCl. The resulting layers were separated and the organic layer was washed with 50 ml of a saturated NaCl solution, dried over Na₂SO₄, filtered and then concentrated in vacuo to provide a yellow oil. This oil was purified using flash chromatography (eluent of 10% EtOAc in hexanes).
Yield: 158 mg of a clear oil (96%).
m.p. 105-107°C.
IR (KBr): 2957, 3500 and 1719 cm⁻¹.
¹H NMR (300MHz, CDCl₃): δ7.20 (m, 3H); 4.75 (t, J=4.8Hz, 1H); 3.69 (s, 3H); 2.88 (m, 1H); 2.50 (dd, J=1.8,13.2Hz, 1H); 2.30 (brd, J=12.1Hz, 1H); 2.06-2.17 (m, 1H); 1.98 (d, J=7.0Hz, 1H); 1.69-1.84 (m, 4H); 1.55 (m, 2H); 1.29 (s, 3H); 1.25 (s, 3H); 1.23 (s, 3H) and 1.17 (s, 3H).
MS(FD): m/e 330(M+).

### Example 5 (Reference compound)

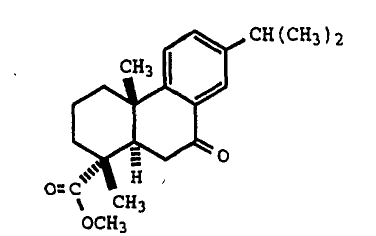

To a solution of 112 mg (0.30 mmol) of the compound of Example 4 in 4 ml of glacial AcOH and 1 ml of H₂O, was added 10 mg (1.0 mmol) of chromium trioxide. The reaction mixture was stirred at room temperature for 1 hour and then partitioned between 50 ml of EtOAc and 50 ml of a saturated NaCl solution. The resultant layers were separated and the organic layer was dried over Na₂SO₄, filtered and then concentrated in vacuo to provide a dark oil which was purified using flash chromatography (eluent of 5% EtOAc in hexanes) to provide an oil which solidified on standing. Yield: 100 mg (90%).
IR (CHCl₃): 2965, 1722, 1675 and 1253 cm⁻¹.
¹H NMR (300MHz, CDCl₃): δ7.88 (d, J=1.8Hz, 1H); 7.42 (dd, J=1.8,8.1Hz, 1H); 7.30 (d, J=8.1Hz, 1H); 3.66 (s, 3H); 2.86-2.98 (m, 1H); 2.66-2.76 (m, 2H); 2.28-2.40 (m, 2H); 1.60-1.90 (m, 5H); 1.35 (s, 3H); 1.26 (s, 6H) and 1.24 (s, 3H).
MS(FD): m/e 329(M+).

| Elemental Analysis for C₂₁H₂₈O₃: | | |
|---|---|---|
| Calcd | C, 76.79; | H, 8.59; |
| Found | C, 76.52; | H, 8.53. |

### Example 6 (Reference compound)

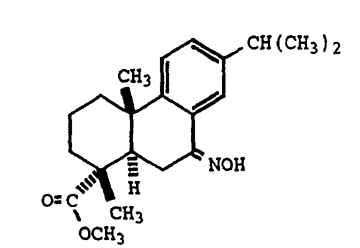

A mixture containing 118 mg (0.36 mmol) of the compound of Example 5, 40 mg (0.58 mmol) of hydroxylamine hydrochloride, 40 mg (0.48 mmol) of NaHCO₃, 1 drop of glacial AcOH, 1.0 ml of H₂O and 15 ml of MeOH was refluxed with a Dean-Stark trap for 5 hours. The reaction mixture was concentrated in vacuo to provide a residue. This residue was partitioned between H₂O and CH₂Cl₂ and the organic layer was dried over Na₂SO₄, filtered and concentrated in vacuo. The crude material was purified using flash chromatography.
Yield: 120 mg (97%).
IR(CHCl₃): 3582, 2962, 1721 and 1261 cm⁻¹.
¹H NMR (300MHz, CDCl₃): δ7.71 (s, 1H); 7.43 (s, 1H); 7.21 (s, 2H); 3.66 (s, 3H); 2.85-2.95 (m,lH); 2.67 (m, 2H); 2.26-2.37 (m, 2H); 1.75 (m, 5H); 1.38 (s, 3H); 1.26 (s, 3H); 1.24 (s, 3H); 1.13 (s, 3H); MS(FD) m/e 343(M+).

### Example 7 (Reference compound)

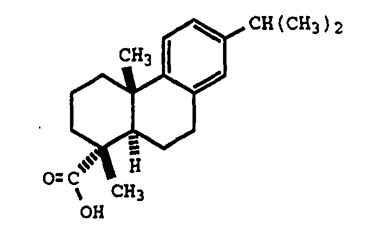

A mixture of 500 mg (1.59 mmol) of the compound of Example 3, 1.0 g (17.8 mmol) of KOH and 20 ml of n-butyl alcohol was refluxed for 16 hours, under N₂. After cooling, the mixture was acidified with 5N HCl and concentrated in vacuo to provide a residue. This residue was suspended in 50 ml of H₂O and filtered. The resulting solid was dissolved in 50 ml of MeOH, filtered and the filtrate was concentrated in vacuo.
Yield: 330 mg of a foam (69%).
m.p. 143-145°C.
IR (KBr): 2958, 1695 and 1279 cm⁻¹.
¹H NMR (300MHz, CDCl₃): δ7.25 (d, J=3Hz, 1H); 7.17 (d, J=8Hz, 1H); 7.00 (dd, J=3.9Hz, 1H); 6.89 (brs, 1H); 2.80-3.00 (m, 3H); 2.20-2.40 (m, 2H); 1.65-1.96 (m, 5H); 1.43-1.60 (m, 2H); 1.29 (s, 3H); 1.24 (s, 3H); 1.22 (s, 3H); 1.21 (s, 3H);
MS(FD) m/e 301(M+).

| Elemental Analysis for C₂₀H₂₈O₂·0.5H₂O: | | |
|---|---|---|
| Calcd | C, 79.01; | H, 9.47; |
| Found | C, 79.19; | H, 9.52. |

### Example 8 (Reference compound)

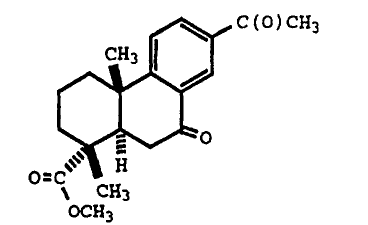

To a cold (O°C) solution of 8.0 g (25.0 mmol) of the compound of Example 3 in 50 ml of acetic anhydride and 38 ml of AcOH, was added 11.0 g (0.11 mmol) of chromium trioxide slowly, under N₂. The reaction mixture was partitioned between EtOAc and brine and the organic layer was dried over Na₂SO₄, filtered and concentrated to provide a yellow oil. This oil was purified using flash chromatography (SiO₂, eluent of 10% EtOAc in hexanes) to provide a solid which was filtered with the aid of hexanes.
Yield: 2.5 g (30.5%).
m.p. 144-145°C.
IR (KBr): 2951, 1725 and 1680 cm⁻¹.
¹H NMR (300MHz, CDCl₃): δ8.55 (d, J=2Hz, 1H); 8.17 (dd, J=2,8Hz, 1H); 7.50 (d, J=8Hz, 1H); 3.66 (s, 3H); 2.75 (m, 2H); 2.64 (s, 3H); 2.37-2.50 (m, 2H); 1.60-1.90 (m, 5H); 1.37 (s, 3H) and 1.29 (s, 3H).
MS(FD): m/e 328 (M+).

| Elemental Analysis for C₂₀H₂₄O₄: | | |
|---|---|---|
| Calcd | C, 73.15; | H, 7.37; |
| Found | C, 72.86; | H, 7.42. |

### Example 9 (Reference compound)

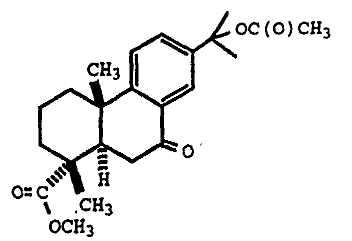

The compound was isolated from the reaction mixture described in Example 8.
Yield: 4.2 g of a white solid (43.5%).
m.p. 130-133°C.
IR (KBr): 2934, 1734, 1720 and 1680 cm⁻¹.
¹H NMR (300MHz, CDCl₃): δ7.98 (d, J=2Hz, 1H); 7.54 (dd, J=2,8Hz, 1H); 7.33 (d, J=8Hz, 1H); 3.66 (s, 3H); 2.75 (m, 2H); 2.30-2.42 (m, 2H); 2.2 (m, 1H); 2.04 (s, 3H); 2.00-2.10 (m, 1H); 1.70-1.90 (m, 3H); 1.76 (s, 6H); 1.35 (s, 3H) and 1.26 (s, 3H).
MS(FD): m/e 386 (M+).

| Elemental Analysis for C₂₃H₃₀O₅: | | |
|---|---|---|
| Calcd | C, 71.48; | H, 7.82; |
| Found | C, 71.75; | H, 8.03. |

### Example 10 (Reference compound)

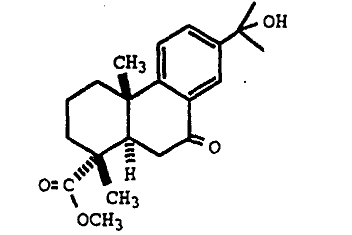

To a solution of 4.14 g (10.7 mmol) of the compound of Example 9 in 40 ml of MeOH, was added 13.4 ml (13.4 mmol) of 1N NaOH. The reaction mixture was refluxed for 2.5 hours, cooled and then partitioned between 200 ml of 0.2N HCl and 200 ml of EtOAc. The resultant layers were separated and the organic layer was washed with 200 ml of brine, dried over Na₂SO₄, filtered and then concentrated in vacuo to provide a dark yellow oil. This oil was purified using flash chromatography (SiO₂, eluent of 15% EtOAc in hexanes).
Yield: 2.9 g of a white foam (80%).
m.p. 57-60°C.
IR(KBr): 3444, 2936, 1727 and 1682 cm⁻¹.
¹H NMR (300MHz, CDCl₃): δ8.06 (d, J=2.2Hz, 1H); 7.74 (dd, J=2.2,8.5Hz, 1H); 7.36 (d, J=8.5Hz, 1H); 3.66 (s, 3H); 2.70 (m, 2H); 2.40 (m, 2H); 1.60-1.90 (m, 6H); 1.60 (s, 3H); 1.55 (s, 3H); 1.35 (s, 3H) and 1.27 (s, 3H).
MS(FD): m/e 344(M+).

| Elemental Analysis for C₂₁H₂₈O₄: | | |
|---|---|---|
| Calcd | C, 73.23; | H, 8.19; |
| Found | C, 73.50; | H, 8.46. |

### Example 11 (Reference compound)

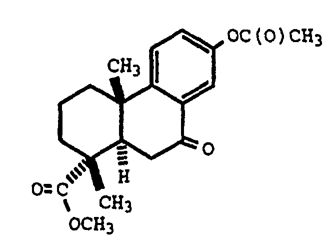

A mixture of 500 mg (1.52 mmol) of the compound of Example 8, 620 mg (1.80 mmol) of 50% m-chloroperbenzoic acid, 5.0 mg (0.03 mmol) of p-toluene sulfonic acid monohydrate and 5 ml of 1,2-dichloroethane was refluxed for 4 hours and then stirred overnight at room temperature. The mixture was diluted with 25 ml of EtOAc and washed sequentially with 25 ml of 10% potassium iodide, 10% sodium thiosulfate, saturated NaHCO₃ and brine, dried over Na₂SO₄, filtered and then concentrated in vacuo. The crude material was purified by radial chromatography (eluent of 25% Et₂O in hexanes).
Yield: 30 mg (6%).
IR(CHCl₃): 3020, 1723 and 1684 cm⁻¹.
¹H NMR (300MHz, CDCl₃): δ7.69 (d, J=3Hz, 1H); 7.39 (d, J=9Hz, 1H); 7.24 (d, J=3Hz, 1H); 3.67 (s, 3H); 2.84 (m, 2H); 2.25-2.42 (m, 2H); 2.31 (s, 3H); 1.60-1.90 (m, 5H); 1.35 (s, 3H) and 1.28 (s, 3H).
MS(FD): m/e 344(M+).

| Elemental Analysis for C₂₀H₂₄O₅: | | |
|---|---|---|
| Calcd | C, 69.75; | H, 7.02; |
| Found | C, 69.77; | H, 6.92. |

### Example 12 (Reference compound)

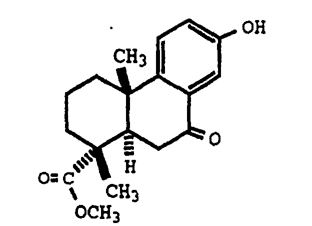

The compound was prepared substantially in accordance with the procedure detailed in Matsumoto et al., Bull. Chem. Soc. Jpn., vol. 61, pages 723-727 (1988), using the compound of Example 8.
Yield: 42%.
IR(CHCl₃): 3389, 2948, 1725, 1670, 1606 cm⁻¹.
¹H NMR (300MHz, CDCl₃): δ7.45 (d, J=3Hz, 1H); 7.27 (d, J=9Hz, 1H); 7.07 (dd, J=3,9Hz, 1H); 5.20 (s, 1H); 3.66 (s, 3H); 2.65-2.80 (m, 2H); 2.27-2.42 (m, 2H); 1.60-1.90 (m, 5H); 1.34 (s, 3H) and 1.25 (s, 3H).
MS(FD): m/e 302(M+).

| Elemental Analysis for C₁₈H₂₂O₄: | | |
|---|---|---|
| Calcd | C, 71.50; | H, 7.33; |
| Found | C, 71.22; | H, 7.19. |

### Example 13 (Reference compound)

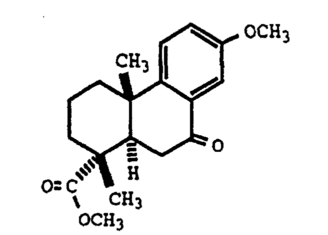

The compound was prepared substantially in accordance with the procedure detailed in Matsumoto et al., Bull. Chem. Soc. Jpn., vol. 61, 723-727 (1988), using the compound of Example 12.
Yield: 86%.
IR(CHCl₃): 2941, 1722, 1677 and 1252 cm⁻¹.
¹H NMR (300MHz, CDCl₃): δ7.48 (d, J=3Hz, 1H); 7.29 (d, J=9Hz, 1H); 7.11 (dd, J=3,9Hz, 1H); 3.84 (s, 3H); 3.66 (s, 3H); 2.70 (m, 2H); 2.30-2.43 (m, 2H); 1.60-1.90 (m, 5H); 1.34 (s, 3H) and 1.25 (s, 3H).
MS(FD): m/e 316(M+).

| Elemental Analysis for C₁₉H₂₄O₄: | | |
|---|---|---|
| Calcd | C, 72.13; | H, 7.65; |
| Found | C, 72.16; | H, 7.35. |

### Example 14 (Reference compounds)

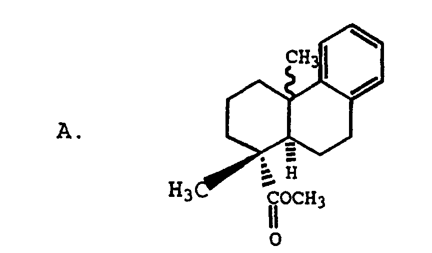

A mixture of 475 mg (1.5 mmol) of the compound of Example 3, 425 mg (3.19 mmol) of anhydrous aluminum chloride in 15 ml of toluene was stirred at room temperature for 2 hours, under N₂. The reaction mixture was partitioned between toluene and 1N HCl. The resultant layers were separated and the organic layer was washed with brine, dried over Na₂SO₄, filtered and concentrated in vacuo to provide an oil. This oil was purified using flash chromatography (SiO₂, eluent of 2% EtOAc in hexanes) to provide an oil which was crystallized from MeOH .
¹H NMR (300MHz, CDCl₃): δ7.00-7.30 (m, 4H); 3.30 (s, 1.5H); 3.28 (s, 1.5H); 2.90 (m, 2H); 2.30 (m, 2H); 2.00 (m, 1H) ; 1.40-1.80 (m, 6H); 1.30 (s, 1.5H); 1.22 (s, 3H) and 1.10 (s, 1.5H).

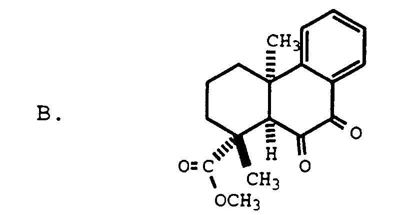

A solution of 285 mg (2.8 mmol) of chromium trioxide in 4 ml of glacial AcOH and 1 ml of H₂O was added dropwise to a solution of 275 mg (1 mmol) of the compound of Example 14A in 5 ml of glacial AcOH. The reaction mixture was stirred at room temperature for 2 hours and then partitioned between EtOAc and brine (twice). The combined organic layers were dried over Na₂SO₄, filtered and then concentrated in vacuo to provide a yellow oil which was purified using flash chromatography (SiO₂, eluent of 5% EtOAc in hexanes).
Yield: 50 mg of a bright yellow solid (17%).
m.p. 121-123°C.
IR(CHCl₃) : 3019, 2954, 1727, 1688 and 1248 cm⁻¹.
¹H NMR (300MHz, CDCl₃): δ8.14 (d, J=8Hz, 1H); 7.70 (7, J=7Hz, 1H); 7.47 (m, 2H); 3.73 (s, 3H); 3.39 (s, 1H); 2.64 (d, J=12Hz, 1H); 2.01-2.11 (m, 1H); 1.40-1.80 (m, 4H); 1.29 (s, 3H) and 0.69 (s, 3H).
MS(FD): m/e 300(M+).

| Elemental Analysis for C₁₈H₂₀O₄: | | |
|---|---|---|
| Calcd | C, 71.98; | H, 6.71; |
| Found | C, 72.10; | H, 6.66. |

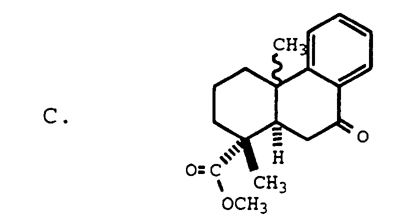

The compound was isolated Example 14B.
Yield: 136 mg of an oil (47.5%).
¹H NMR (300MHz, CDCl₃): δ8.01 (m, 1H); 7.55 (m, 1H); 7.30 (m, 2H); 3.30 (s, 1.5H); 3.28 (s, 1.5H); 3.10 (dd, J=4,12Hz, 0.5H): 2.70 (m, 1.5H); 2.40 (m, 2H); 1.40-1.90 (m, 5H); 1.30 (s, 1.5H); 1.28 (s, 1.5H); 1.23 (s, 1.5H); 0.65 (s, 1.5H).

### Example 15 (Intermediates)

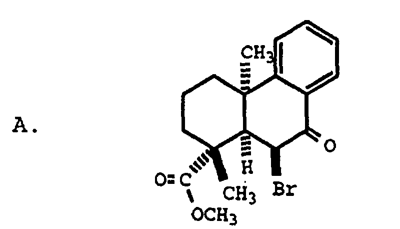

A solution of 0.9 ml (17 mmol) of bromine in 30 ml of anhydrous Et₂O was added to a solution of 3.8 g (13.3 mmol) of the compound of Example 14C in 200 ml of anhydrous Et₂O, dropwise. The reaction mixture was stirred at room temperature for 1 hour and then washed sequentially with H₂O, a saturated NaHCO₃ solution and 19% sodium thiosulfate, dried over Na₂SO₄, filtered and concentrated in vacuo to provide a residue. This residue was purified using flash chromatography (eluent of 3:2 CH₂Cl₂/hexanes).
Yield: 1.2 g of yellowish oil (25%).
¹H NMR (300MHz, CDCl₃): δ8.00 (dd, J=2,5Hz, 1H); 7.60 (dt, J=2,5Hz, 1H); 7.40 (m, 2H); 4.60 (s, 1H); 3.78 (s, 3H); 3.25 (s, 1H) 2.50 (d, J=7Hz, 1H); 1.60-1.90 (m, 5H); 1.60 (s 3H); 0.57 (s, 3H).

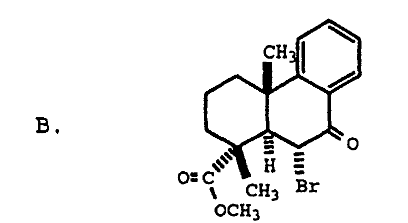

The compound was isolated from Example 15A.
Yield: 1.2 g of an oil (25%).
¹H NMR (300MHz, CDCl₃) : δ8.01 (d, J=6Hz, 1H); 7.60 (m, 1H) ; 7.40 (m, 2H); 5.00 (d, J=9Hz, 1H); 3.65 (s, 3H); 3.23 (d, J=9Hz, 1H); 2.60 (m, 1H); 2.38 (d, J=7Hz, 1H); 1.80 (m, 4H); 1.52 (s, 3H) and 1.25 (s, 3H).

### Example 16 (Reference compound)

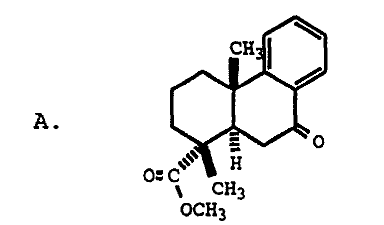

A mixture of 960 mg (2.6 mmol) of the compound of Example 15B, 4.0 g (61.2 mmol) of zinc dust, 2.0 g (24.4 mmol) of NaOAc and 50 ml of glacial AcOH was refluxed for 1 hour, under N₂. After cooling, the reaction mixture was filtered and the filtrate was partitioned between Et₂O and brine. The resultant layers were separated and the organic layer was dried over Na₂SO₄, filtered and then concentrated in vacuo. The crude material was purified using flash chromatography (SiO₂, eluent of 10% Et₂O in hexanes).
Yield: 500 mg of an oil (67.2%).
IR(CHCl₃): 3028, 1722, 1679, 1258 cm⁻¹.
¹H NMR (300MHz, CDCl₃): δ8.01 (d, J=8Hz, 1H); 7.54 (7, 6Hz, 1H); 7.29 (m, 2H); 3.67 (s, 3H); 2.74 (dd, J=3,7Hz, 2H); 2.30-2.45 (m, 2H); 1.60-1.90 (m, 5H); 1.36 (s, 3H) and 1.28 (s, 3H).
MS(FD): m/e 286(M+).

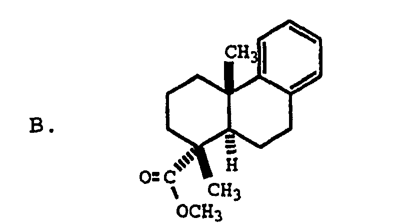

The compound was isolated from Example 16A.
Yield: 150 mg (21%).

### Example 17 (Reference compound)

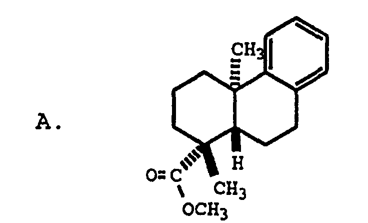

A solution of 1.54 g (5.66 mmol) of the compound of Example 14A and 1.5 g of 10% Pd/C in 150 ml of triethyleneglycol dimethyl ether was refluxed for 3 hours, under N₂. After cooling, the mixture was filtered and the filtrate was partitioned between EtOAc and brine (three times). The resultant layers were separated and the combined organic layers were dried over Na₂SO₄, filtered and the concentrated in vacuo to provide 1.5 g of a residue. A fraction of this residue (300 mg) was purified by chromatotron (eluent of 2% CH₂Cl₂ in hexanes initially, followed by the addition to the mobile phase of 50 ml of CH₂Cl₂ after 200 ml elution, and finally 4 ml of EtOAc after 300 ml had eluted).
Yield: 29 mg
¹H NMR (300MHz, CDCl₃): δ7.00-7.30 (m, 4H); 3.63 (s, 3H); 2.83 (m, 2H); 2.30 (m, 3H); 2.00 (m, 2H); 1.30-1.70 (m, 3H); 1.25 (s, 3H); 1.10 (m, 1H); 1.02 (s, 3H).

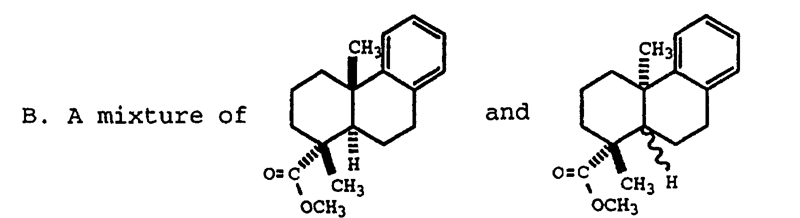

The compounds were isolated from Example 17A.
Yield: 216 mg.

### Example 18 (Reference compound)

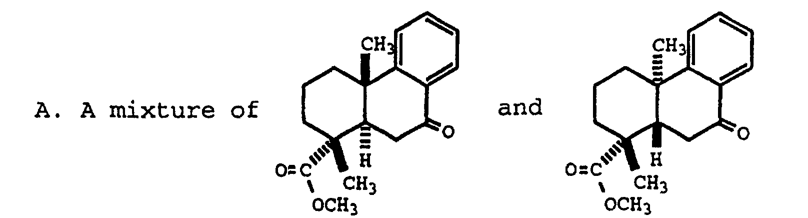

The compounds were prepared substantially in accordance with the procedure detailed in Example 14B, using a solution of 325 mg (1.2 mmol) of the unpurified residue from Example 17A in 5 ml of glacial AcOH The crude material was purified using flash chromatography (SiO₂, eluent of 15% Et₂O in hexanes).
Yield: 62 mg of an oil.

### Example 19

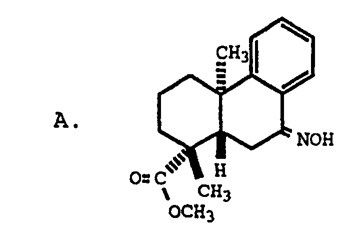

The compound was prepared substantially in accordance with the procedure detailed in Example 6, using a solution containing 140 mg (0.49 mmol) of the compounds of Example 18A in 10 ml of MeOH. The crude material was purified using flash chromatography (SiO₂, eluent of 20% Et₂O in hexanes).
Yield: 38 mg of a solid (26%).
m.p. 139-141°C.
IR(CHCl₃): 3584, 3020 and 1720 cm⁻¹.
¹H NMR (300MHz, CDCl₃): δ7.92 (d, J=7.7Hz, 1H); 7.56 (s, 1H); 7.30 (m, 2H); 7.20 (m, 1H); 3.72 (s, 3H); 3.44 (dd, J=4.0,18.4Hz, 1H); 3.12 (dd, J=14.0,18.7Hz, 1H); 2.35 (m, 2H); 2.03 (m, 1H); 1.50-1.80 (m, 3H); 1.32 (s, 3H); 1.10 (m, 1H) and 1.02 (s, 3H).
MS(FD): m/e 301(M+).

| Elemental Analysis for C₁₈H₂₃NO₃: | | | |
|---|---|---|---|
| Calcd | C, 71.74; | H, 7.69; | N, 4.65; |
| Found | C, 71.97; | H, 7.77; | N, 4.39. |

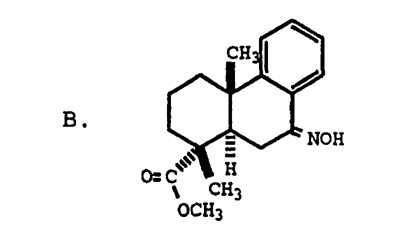

The compound was isolated from Example 19A.
Yield: 38 mg of a resin (26%).
IR(CHCl₃): 3583, 3027, 2935, 1721 and 1263 cm⁻¹.
¹H NMR (300MHz, CDCl₃): δ7.85 (d, J=8.1Hz, 1H); 7.43 (s, 1H); 7.10-7.20 (m, 3H); 3.66 (s, 3H); 2.67 (d, J=8.8Hz, 2H); 2.28-2.37 (m, 2H); 1.76 (m, 4H); 1.39 (s, 3H); 1.26 (m, 1H) and 1.14 (s, 3H).
MS(FD): m/e 301(M+).

### Example 20

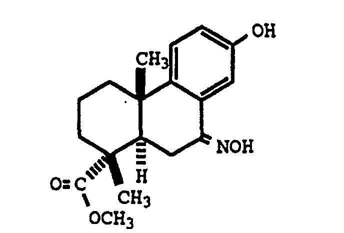

The compound was prepared substantially in accordance with the procedure detailed in Example 6, using the compound of Example 12.
IR(KBr): 3393, 2932, 1727 and 1702 cm⁻¹.
¹H NMR (300MHz, CDCl₃): δ8.00 (brs, 1H); 7.29 (d, J=3Hz, 1H); 7.16 (d, J=9Hz, 1H); 6.85 (dd, J=3,9Hz, 1H); 5.25 (brs, 1H); 3.68 (s, 3H); 2.65 (m, 2H); 2.20-2.40 (m, 2H); 1.55-2.04 (m, 5H); 1.40 (s, 3H) and 1.10 (s, 3H).
MS(FD): m/e 318(M+).

| Elemental Analysis for C₁₈H₂₃O₄: | | | |
|---|---|---|---|
| Calcd | C, 68.12; | H, 7.30; | N, 4.41; |
| Found | C, 67.95; | H, 7.46; | N, 4.12. |

### Example 21

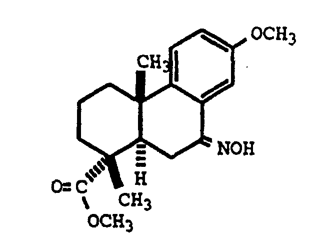

The compound was prepared substantially in accordance with the procedure detailed in Example 6, using the compound of Example 13.
Yield: 93%.
IR(KBr): 3421, 2936, 1727 cm⁻¹.
¹H NMR (300MHz, CDCl₃): δ7.37 (d, J=3Hz, 1H); 7.29 (s, 1H) ; 6.92 (d, J=9Hz, 1H); 6.91 (dd, J=3,9Hz, 1H); 3.81 (s, 3H); 3.66 (s, 3H); 2.65 (m, 2H); 2.25-2.65 (m, 2H); 1.60-1.80 (m, 5H); 1.38 (s, 3H) and 1.11 (s, 3H).
MS(FD): m/e 331(M+).

| Elemental Analysis for C₁₉H₂₅NO₄: | | | |
|---|---|---|---|
| Calcd | C, 68.86; | H, 7.60; | N, 4.23' |
| Found | C, 69.10; | H, 7.83; | N, 4.23. |

### Example 22 (Intermediate)

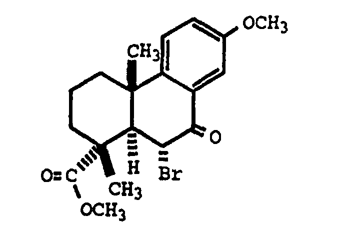

The compound was prepared substantially in accordance with the procedure detailed in Example 15A, using the compound of Example 13.
Yield: 74%
m.p. 146-148°C.
IR(KBr): 2900, 1725 and 1679 cm⁻¹.
¹H NMR (300MHz, CDCl₃): δ7.46 (d, J=3Hz, 1H); 7.28 (d, J=9Hz, 1H); 7.14 (dd, J=3,9Hz, 1H); 4.98 (d, J=13Hz, 1H); 3.85 (s, 3H); 3.65 (s, 3H); 3.20 (d, J=13Hz, 1H); 2.35 (m, 1H); 1.70-1.90 (m, 5H); 1.50 (s, 3H); 1.26 (s, 3H).
MS(FD): m/e 397 (M+).

| Elemental Analysis for C₁₉H₂₃BrO₄: | | |
|---|---|---|
| Calcd | C, 57.73; | H, 5.86; |
| Found | C, 57.78; | H, 6.06. |

### Example 23 (Reference compound)

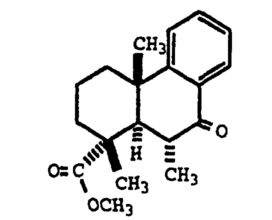

To a stirring solution of 70 mg (0.245 mmol) of the compound of Example 16A in 20 ml of anhydrous THF, was added 32 mg (0.80 mmol) of 60% NaH on mineral oil, under N₂. The resultant mixture was stirred 15 minutes followed by the addition of 0.15 ml (2.25 mmol) of iodomethane, via syringe. The reaction mixture was stirred for 3 hours, quenched by the dropwise addition of H₂O and then partitioned between Et₂O and H₂O. The resultant layers were separated and the organic layer was dried over Na₂SO₄, filtered and concentrated in vacuo to provide a residue which was purified by radial chromatography (eluent of 10% Et₂O in hexanes).
Yield: 16 mg of a clear oil (22%).
IR(CHCl₃): 3692, 3022, 2950, 1724 and 1678 cm⁻¹.
¹H NMR (300MHz, CDCl₃): δ7.95 (d, J=7.7Hz, 1H); 7.54 (s, J=8.8Hz, 1H); 7.25 (m, 2H); 3.63 (s, 3H); 2.80 (m, 2H); 2.39 (d, J=7.7Hz, 1H); 1.70-2.05 (m, 5H); 1.43 (s, 3H); 1.28 (s, 3H) and 1.13 (d, J=6.2Hz, 3H).
MS(FD): m/e 300 (M+).

| Elemental Analysis for C₁₉H₂₄O₃: | | |
|---|---|---|
| Calcd | C, 75.97; | H, 8.05; |
| Found | C, 75.80; | H, 7.96. |

### Example 24

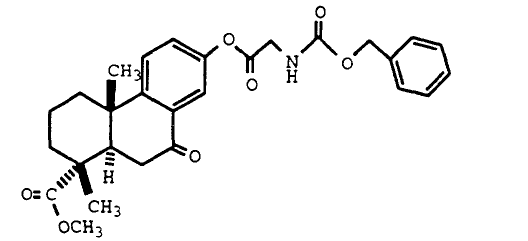

A mixture of 50 mg (0.165 mmol) of the compound of Example 12, 35 mg (0.165 mmol) of carbobenzyloxyglycine, 35 mg (0.170 mmol) of 1,3-dicyclohexylcarbodiimide (DCC), 2 mg (0.0165 mmol) of 4-dimethylaminopyridine in 25 ml of anhydrous Et₂O was stirred for 16 hours resulting in the formation of a solid. This solid was removed by filtration and the filtrate was washed sequentially with H₂O, a 5% AcOH solution and brine. The combined organic layers were dried over Na₂SO₄, filtered and then concentrated in vacuo to provide a resin. This resin was purified using flash chromatography (SiO₂, eluent of 20% Et₂O in hexanes).
Yield: 54 mg of a light yellow solid (66%).
IR(KBr): 3330, 2934, 1779, 1725, 1685 cm⁻¹.
¹H NMR (300MHz, CDCl₃): δ7.70 (s, 1H); 7.36 (m, 8H); 6.20 (s, 2H); 4.26 (d, J=6Hz, 2H); 3.67 (s, 3H); 2.73 (dd, J=3,6Hz, 2H); 2.40 (m, 2H); 1.50-2.00 (m, 5H); 1.35 (s, 3H); 1.28 and (s, 3H).
MS(FD): m/e 494 (M+).

### Example 25

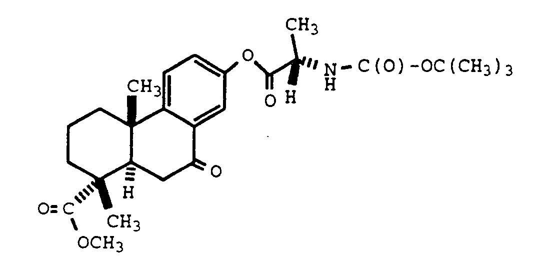

The compound was prepared substantially in accordance with the procedure detailed in Example 24, using the compound of Example 12 and N-t-butoxycarbonyl-L-alanine .
Yield: 96%.
IR(CHCl₃): 3444, 2938, 1763, 1714 cm⁻¹.
¹H NMR (300MHz, CDCl₃): δ7.69 (d, J=2.6Hz, 1H); 7.41 (d, J=8.5Hz, 1H); 7.30 (d, J=2.6Hz, 1H); 5.10 (brm, 1H); 4.55 (brm, 1H); 3.65 (s, 3H); 2.73 (dd, J=3.3, 6.6Hz, 2H); 2.32-2.42 (m, 2H); 1.70-2.20 (m, 5H); 1.55 (d, J=7.0Hz, 3H); 1.47 (s, 6H); 1.43 (s, 3H); 1.35 (s, 3H) and 1.28 (s, 3H).
MS(FD): m/e 474 (M+).

### Example 26

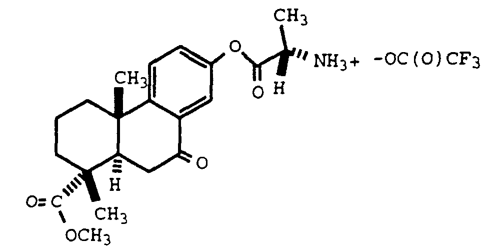

A mixture of 100 mg (0.21 mmol) of the compound of Example 25 in 2 ml of CH₂Cl₂ was added to 2 ml of a 1:1 CH₂Cl₂/CF₃COOH mixture. After stirring for 1 hour, the reaction mixture was concentrated in vacuo. The resultant residue was concentrated repetitively in Et₂O until a foam was obtained.
Yield: 85 mg (83%).
IR(CHCl₃): 2941, 1715, 1711 and 1682 cm⁻¹.
¹H NMR (300MHz, CDCl₃): δ7.65 (m, 2H); 7.41 (d, J=8.1Hz, 1H); 4.30 (m, 1H); 3.62 (s, 3H); 2.63 (m, 2H); 2.25-2.40 (m, 2H); 1.60-1.90 (m, 5H); 1.34 (s, 3H) and 1.24 (s, 3H); 1.22 (d, J=7.0Hz, 3H).
MS(FD): m/e 374, free base (M+).

### Example 27

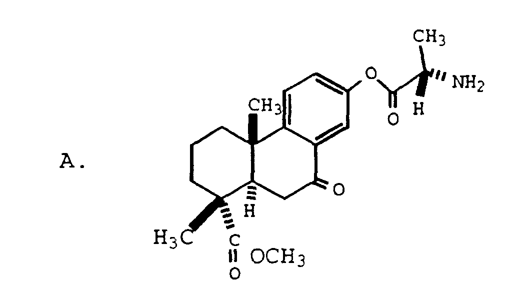

To a solution of 550 mg (1.0 mmol) of the compound of Example 25 in 10 ml of CH₂Cl₂, was added 3 ml (39 mmol) of CF₃COOH. After stirring for 1 hour, the reaction mixture was concentrated in vacuo to provide a foam. This foam was dissolved in 20 ml of CH₂Cl₂ followed by the addition of 0.17 ml (1.2 mmol) of Et₃N. The resultant layers were separated and the organic layer was washed with H₂O dried over Na₂SO₄, filtered and concentrated in vacuo to provide a foam.

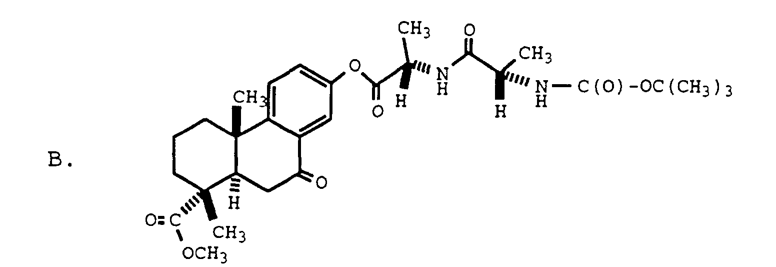

The compound was prepared substantially in accordance with the procedure detailed in Example 24, using the compound of Example 27A and 230 mg (1.2 mmol) of N-t-butyloxycarbonyl-L-alanine
Yield: 255 mg (47%).
IR(KBr): 3334, 2938, 1770, 1725 and 1687 cm⁻¹.
¹H NMR (300MHz, CDCl₃): δ7.69 (d, J=3Hz, 1H); 7.41 (d, J=9Hz, 1H); 7.29 (d, J=3Hz, 1H); 6.70 (m, 1H); 5.00 (m, 1H); 4.80 (m, 1H); 4.20 (m, 1H); 3.67 (s, 3H); 2.73 (m, 2H); 2.36 (m, 2H); 1.60-1.90 (m, 5H); 1.58 (d, J=7Hz, 3H); 1.45 (s, 9H); 1.38 (d, J=7Hz, 3H); 1.35 (s, 3H) and 1.27 (s, 3H). MS(FD): m/e 544 (M+).

| Elemental Analysis for C₂₉H₄₀N₂O₈·0.75H₂O: | | | |
|---|---|---|---|
| Calcd | C, 62.40; | H, 7.43; N, | 5.02; |
| Found | C, 62.30; | H, 7.24; N, | 4.77. |

### Example 28

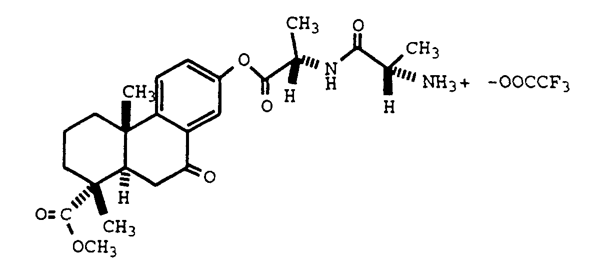

The compound was prepared substantially in accordance with the procedure detailed in Example 26, using the compound of Example 27B.
Yield: 95%..
IR(KBr): 2950, 1770 and 1681 cm⁻¹.
¹H NMR (300MHz, CDCl₃) : δ8.17 (brs, 3H); 7.60 (s, 1H); 7.40 (d, J=2.6Hz, 1H); 7.27 (m, 1H); 4.65 (brs, 1H); 4.40 (brs, 1H); 3.70 (d, J=2Hz, 1H); 3.62 (s, 3H); 2.70 (m, 7H); 2.35 (m, 2H); 1.50-1.85 (m, 6H); 1.32 (s, 3H) and 1.23 (s, 3H). MS(FD): m/e 445 free base (M+).

### Example 29

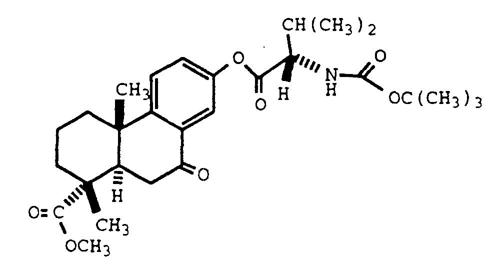

The compound was prepared substantially in accordance with the procedure detailed in Example 24, using the compound of Example 12 and N-t-butoxycarbonyl-L-valine.
Yield: 47%.
IR(KBr): 3377, 2969, 1766, 1724 and 1688 cm⁻¹.
¹H NMR (300MHz, CDCl₃): δ7.68 (d, J=2.6Hz, 1H); 7.41 (d, J=8.8Hz, 1H); 7.29 (d, J=2.6Hz, 1H); 5.06 (brm, 1H); 4.45 (brm, 1H); 3.65 (s, 3H); 2.74 (dd, J=3.3,7.0Hz, 2H); 2.30-2.43 (m, 1H); 1.70-1.90 (m, 6H); 1.48 (s, 9H); 1.35 (s, 3H); 1.28 (s, 3H); 1.09 (d, J=6.6Hz, 3H); 1.02 (d, J=7.0Hz, 3H). MS(FD): m/e 502 (M+).

| Elemental Analysis for C₂₈H₃₉NO₇: | | | |
|---|---|---|---|
| Calcd | C, 67.04; | H, 7.84; | N, 2.79; |
| Found | C, 66.82; | H, 7.73; | N, 2.58. |

### Example 30

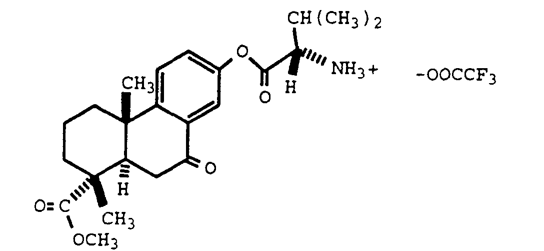

The compound was prepared substantially in accordance with the procedure detailed in Example 26, using the compound of Example 29.
Yield: 99% yield.
IR(KBr): 3068, 2943, 1758, 1722 and 1668 cm⁻¹.
¹H NMR (300MHz, CDCl₃): δ7.68 (m, 1H); 7.40 (m, 2H); 4.08 (d, J=4.8Hz, 1H); 3.65 (s, 3H); 2.70 (m, 2H); 2.30-2.40 (m, 2H); 1.70-1.90 (m, 6H); 1.34 (s, 3H); 1.26 (s, 3H); 1.16 (d, J=2.9Hz, 3H) and 1.14 (d, J=2.9Hz, 3H).
MS(FD): m/e free base 402 (M+).

| Elemental Analysis.for C₂₅H₃₂NO₇: | | | |
|---|---|---|---|
| Calcd | C, 58.25; | H, 6.26; | N, 2.72; |
| Found | C, 57.98; | H, 6.32; | N, 2.64. |

### Example 31

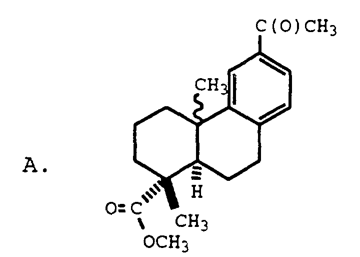

A mixture of 4.0 g (14.7 mmol) of the compound of Example 14A and 1.2 ml (16.9 mmol) of acetyl chloride in 60 ml of carbon disulfide to a suspension of 2.6 mg (19.5 mmol) of anhydrous aluminum chloride in 100 ml of carbon disulfide, via dropping funnel. The reaction mixture was refluxed for 1 hour and then the carbon disulfide was removed by downward distillation. The resultant residue was cautiously quenched by the addition of 100 ml of 0.2N HCl. The desired compound was extracted using 100 ml of CH₂Cl₂, dried over Na₂SO₄, filtered and then concentrated in vacuo to provide a dark red oil. This oil was purified using flash chromatography (SiO₂, eluent of 20% Et₂O in hexanes).
Yield: 1.7 g of an oil (87% based on recovered starting material).
¹H NMR (300MHz, CDCl₃): δ7.90 (d, J=4Hz, 1H); 7.75 (d, J=4Hz, 0.5H); 7.63 (d, J=4Hz, 0.5H); 7.37 (d, J=6Hz, 0.5H); 7.10 (d, J=6Hz, 0.5H); 3.70 (s, 1.5H); 3.68 (s, 1.5H); 2.92 (m, 2H); 2.60 (s, 3H); 2.00-2.50 (m, 3H); 1.40-1.98 (m, 6H); 1.29 (s, 1.5H); 1.26 (s, 1.5H); 1.24 (s, 1.5H) and 1.10 (s, 1.5H).

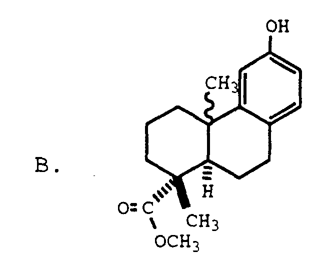

A mixture of 1.7 g (5.4 mmol) of the compound of Example 31A, 1.9 g (5.5 mmol) of 50% 3-chloroperoxybenzoic acid, 18 mg (0.095 mmol) of p-toluene sulfonic acid monohydrate in 25 ml of 1,2-dimethyoxyethane was refluxed for 3 hours, under N₂. After cooling, the reaction mixture was diluted with Et₂O and washed sequentially with 10% potassium iodide, 10% sodium thiosulfate, a saturated NaHCO₃ solution and brine, dried over Na₂SO₄, filtered and then concentrated to provide a resin which was dissolved in 25 ml of MeOH and 10 ml of H₂O containing 1.6 g (19.0 mmol) of NaHCO₃. The mixture was refluxed for 1.5 hours, cooled, filtered and concentrated in vacuo to provide a residue which was partitioned between H₂O and Et₂O. The resulting layers were separated and the organic layer was washed sequentially with 1N HCl and brine, dried over Na₂SO₄, filtered and concentrated in vacuo
Yield: 1.55 g (99%).
¹H NMR (300MHz, CDCl₃): δ6.85 (m, 1H); 6.70 (d, J=6Hz, 1H); 6.55 (dd, J=6Hz, 1H); 3.63 (s, 3H); 2.80 (m, 2H); 1.90-2.30 (m, 3H); 1.40-1.88 (m, 6H); 1.25 (s, 1.5H); 1.20 (s, 1.5H); 1.17 (s, 1.5H) and 1.02 (s, 1.5H).

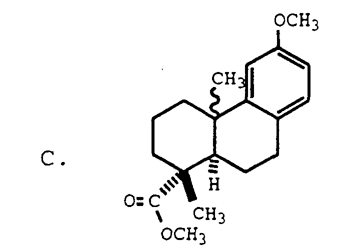

To a suspension of 1.55 g (5.4 mmol) of the compound of Example 31B and 275 mg (6.87 mmol) of 60% NaH on mineral oil in 50 ml of anhydrous DMF, was added 0.5 ml (7.50 mmol) of idodomethane, under N₂. The reaction mixture was stirred for 1 hour and then cautiously quenched by the dropwise addition of brine. The reaction mixture was partitioned between Et₂O and brine. The resultant layers were separated and the organic layer was dried over Na₂SO₄, filtered and concentrated in vacuo to provide a residue which was purified using flash chromatography (SiO₂, eluent of 20% Et₂O in hexanes).
Yield: 1.3 g of a clear light yellow oil (88.5% based on recovered starting material).
¹H NMR (300MHz, CDCl₃): δ7.00 (d, J=6Hz, 1H); 6.82 (m, 1H); 6.70 (m, 1H); 3.80 (s, 3H); 3.70 (s, 3H); 2.80 (m, 2H); 2.00-2.40 (m, 3H); 1.40-1.90 (m, 6H); 1.25 (s, 1.5H); 1.20 (s, 3H); 1.10 (s, 1.5H).
Note: The reaction mixture also contained 200 mg of the compound of Example 31A.

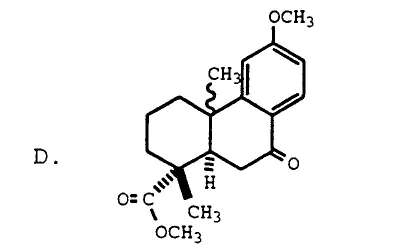

The compound was prepared substantially in accordance with the procedure detailed in Example 14B, using 1.3 g (4.3 mmol) of the compound of Example 31C. The crude material was purified using flash chromatography (SiO₂, eluent of 20% Et₂O in hexanes).
Yield: 820 mg of an oil (60.5%).
¹H NMR (300MHz, CDCl₃): δ8.00 (m, 1H); 6.80 (m, 2H); 3.87 (s, 1.5H); 3.85 (s, 1.5H); 3.63 (s, 1.5H); 3.61 (s, 1.5H); 3.01 (dd, J=5, 12Hz, 0.5H); 2.70 (m, 1.5H); 2.40 (m, 2H); 1.40-1.95 (m, 5H); 1.32 (s, 1.5H); 1.30 (s, 1.5H); 1.22 (s, 1.5H); 0.65 (s, 1.5H).

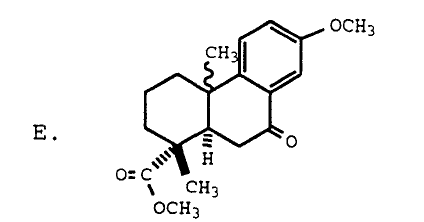

The compound was isolated from Example 31D.
Yield: 35.4 mg of an oil.

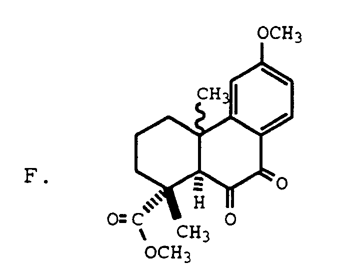

The compound was isolated from Example 31D.
Yield: 150 mg of an oil (10.6%).
¹H NMR (300MHz, CDCl₃): δ8.17 (d, J=6Hz, 1H); 6.95 (m, 1H); 6.80 (m, 1H); 3.90 (s, 3H); 3.70 (s, 3H); 3.36 (s, 0.5H); 2.50 (m, 1.5H); 1.80-2.10 (m, 1H); 1.42-1.80 (m, 4H); 1.40 (s, 1.5H); 1.20 (s, 1.5H); 1.17 (s, 1.5H); 0.65 (s, 1.5H).

### Example 31G-deleted

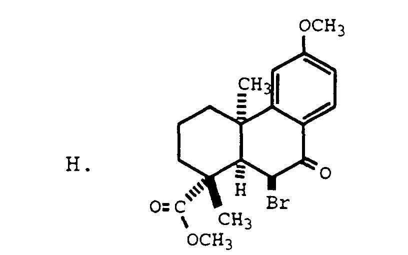

The compound was prepared substantially in accordance with the procedure detailed in Example 15A, using 975 mg (3.08 mmol) of the compound of Example 31D. The crude material was purified using flash chromatography (SiO₂, eluent of 15% of Et₂O in hexanes).
Yield: 532.6 mg (44%).
¹H NMR (300MHz, CDCl₃): δ8.03 (d, J=6Hz, 1H); 6.81 (m, 2H); 4.50 (s, 1H); 3.90 (s, 3H); 3.75 (s, 3H); 3.21 (s, 1H); 2.40 (m, 1H); 1.60-2.00 (m, 5H); 1.58 (s, 3H) and 0.60 (s, 3H).

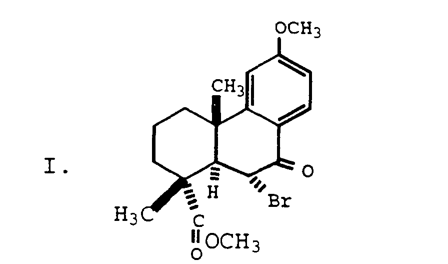

The compound was isolated from Example 31H.
Yield: 465.1 mg of an oil (38%).
¹H NMR (300MHz, CDCl₃): δ8.00 (d, J=6Hz, 1H); 6.85 (m, 2H); 4.97 (d, J=9Hz, 1H); 3.90 (s, 3H); 3.62 (s, 3H); 3.22 (d, J=9Hz, 1H); 2.30 (m, 1H); 1.80 (m, 5H); 1.48 (s, 3H) and 1.28 (s, 3H).

### Example 31J-deleted

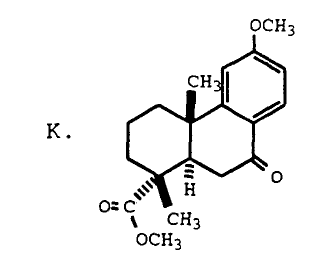

The compound was prepared substantially in accordance with the procedure detailed in Example 16, using 465 mg (1.17 mmol) of the compound of Example 31I.
Yield: 328 mg of a clear oil (89%).
IR(CHCl₃): 2942, 1721, 1667 and 1596 cm⁻¹.
¹H NMR (300MHz, CDCl₃): δ8.01 (d, J=9.2Hz, 1H); 6.85 (s, 1H); 6.81 (d, J=2.6Hz, 1H); 3.90 (s, 3H); 3.66 (s, 3H); 2.70 (m, 2H); 2.30 (m, 2H); 1.60-1.90 (m, 5H); 1.35 (s, 3H) and 1.26 (s, 3H).
MS(FD): m/e 316 (M+).

### Example 32

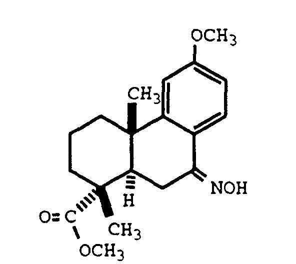

The compound was prepared substantially in accordance with the procedure detailed in Example 6, using the compound of Example 31K.
Yield: 61%.
m.p. 162-165°C.
IR(CHCl₃) : 3018, 2951, 1720 and 1600 cm⁻¹.
¹H NMR (300MHz, CDCl₃): δ7.81 (d, J=6Hz, 1H); 6.82 (m, 3H); 3.90 (s, 3H); 3.70 (s, 3H); 2.62 (d, J=6Hz, 2H); 2.20-2.43 (m, 2H); 1.60-1.90 (m, 5H); 1.40 (s, 3H) and 1.10 (s, 3H). MS(FD): m/e 331 (M+).

| Elemental Analysis for C₁₉H₂₅NO₄: | | | |
|---|---|---|---|
| Calcd | C, 68.86; | H, 7.60; | N, 4.23; |
| Found | C, 68.79; | H, 7.42; | N, 4.33. |

As noted above, the compounds of the present invention are useful for inhibiting an envelope virus that undergoes hemagglutinin-mediated fusion with a host cell. Thus, the claimed compounds may be used to treat or prevent a viral infection where the virus is an envelope virus that undergoes hemagglutinin-mediated fusion which comprises administering to an virus-infected cell, a cell susceptible to infection or a mammal in need thereof an effective amount of a compound of formula I or a pharmaceutically acceptable salt thereof. The claimed compounds may also be used to inhibit viral replication in an envelope virus that undergoes hemagglutinin-mediated fusion which comprises administering to a virus-infected cell, a cell susceptible to infection or a mammal in need thereof, an effective amount of a compound of formula I or a pharmaceutically acceptable salt thereof.

The term "effective amount" as used herein, means an amount of a compound of the present invention which is capable of inhibiting the hemagglutinin mediated fusion of the virus with the host cell. The inhibition contemplated by the present method includes both therapeutic and prophylactic treatment, as appropriate. The specific dose of compound administered according to this invention to obtain therapeutic and/or prophylactic effects will, of course, be determined by the particular circumstances surrounding the case, including, for example, the compound administered, the route of administration, the condition being treated and the individual being treated. A typical daily dose (administered in single or divided doses) will contain a dosage level of from about 0.01 mg/kg to about 50 mg/kg of body weight of an active compound of this invention. Preferred daily doses generally will be from about 0.05 mg/kg to about 20 mg/kg and ideally from about 0.1 mg/kg to about 10 mg/kg.

The compounds can be administered by a variety of routes including oral, rectal, transdermal, subcutaneous, intravenous, intramuscular and intranasal. The compounds of the present invention are preferably formulated prior to administration. Therefore, another embodiment of the present invention is a pharmaceutical formulation comprising an effective amount of a compound of formula I or a pharmaceutically acceptable salt thereof and a pharmaceutically acceptable carrier, diluent or excipient therefor.

The active ingredient in such formulations comprises from 0.1% to 99.9% by weight of the formulation. By "pharmaceutically acceptable" it is meant that the carrier, diluent or excipient is compatible with the other ingredients of the formulation and not deleterious to the recipient thereof.

The present pharmaceutical formulations are prepared by known procedures using known and readily available ingredients. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), ointments containing, for example, up to 10% by weight of the active compound, soft and hard gelatin capsules, suppositories, sterile injectable solutions, sterile packaged powders and the like.

The following experiments were carried out to demonstrate the ability of the compounds of the present invention to inhibit influenza.

### In vitro CPE/XTT Assay

MDCK cells were dispersed in a microtiter plate (96 wells) at 10,000 cells per well with Medium 199 containing Earl's balanced salt solution (EBSS), 1% fetal bovine serum (FBS), penicillin (100 units/ml) and streptomycin (100 µg/ml). After standing overnight at 37°C in a carbon dioxide (CO₂) incubator, the MDCK cells were infected with ∼0.1 moi (mutiplicity of infection) of influenza virus (i.e. A/Kawasaki/89 or B/Hong Kong and B/Great Lakes) at 0.03 moi. After allowing the virus to adsorb to the cells for 1-2 hours, medium containing serial dilutions of drug or medium alone was added to the wells. The resultant mixtures were incubated for 2-3 days (until extensive cpe was apparent in medium alone wells). The antiviral effect of a test compound was assessed by performing the following XTT assay.

A fresh solution (0.4 mg/ml) of XTT [2,3-bis(methoxy-4-nitro-5-sulfophenyl)-2H-tetraazolium-5-carboxanilide, inner salt, sodium salt] in warm medium without FBS was prepared. For each 5 ml of the XTT solution, 25 µl of 5mM PMS (phenazine methosulfate) in phosphate buffer saline was added. After withdrawing the cultured supernatant, 100 µl of the freshly prepared XTT/PMS mixture was added to each of the microtiter wells. The wells were then incubated at 37°C (under CO₂) for 3-4 hours or until color change is prominent. The absorbance at 450 nm (ref. 650 nm) was read in a spectrophotometer. The concentration of test compound required to cause 50% cytotoxic effect (TC₅₀) relative to a control with no drug and no virus present and which inhibits the development of virus cytopathic effect (cpe) by 50% (IC₅₀) or 90% (IC₉₀) was determined from the linear portion of each dose response curve.

Using this CPE/XTT assay, the IC₅₀ of the compounds of formula I was determined to be in the range of 1.0-2.4 µg/ml for influenza A/Kawasaki/89 and in the range of 8-47 µg/ml for influenza B/Lee.

### Plaque Reduction Assay

Susceptible MDCK cells were grown in 6 well tissue culture treated cluster plates at 1x10⁶ cells/well in Minimum 199 with 1 percent fetal bovine serum, penicillin (100 units/ml) and streptomycin (100 µg/ml). After overnight incubation at 37°C, the growth medium was removed and 0.2 ml/well of an appropriate dilution of virus was added. After adsorption for 1-2 hour at room temperature, the infected cell sheet was overlaid with equal parts of 1.5% sterile agarose solution and a twofold concentration of medium 199 (with 2% fetal bovine serum, 100 units/ml of penicillin and 100 µg/ml streptomycin) containing varying concentrations of compounds.

The compounds were dissolved in DMSO at a concentration of 20 mg/ml and an aliquot was diluted to the desired concentration in DMSO and then added to the agar medium mixture. The plates were incubated in a CO₂ incubator at 37°C until the DMSO control wells contained plaques of optimal size. Then, a solution containing 10 percent formalin and 2 percent sodium acetate was added to each well to inactivate the virus and fix the cell sheet to the plastic surface. The fixed cell sheets were stained with 0.5 percent crystal violet and the plaques were counted. Results from duplicate wells at each concentration were averaged and compared with DMSO control wells. The inhibition of plaque formation by 50 or 90 percent (IC₅₀ or IC₉₀) was calculated from the linear region of the inhibition concentration curve using the method of Reed and Muench, Am. J. Hyg., vol. 27, pages 493-497 (1958).

Using this plaque reduction assay, the IC₅₀ of the compounds of formula I was determined to be in the range of 0.02-100 µg/ml for influenza A/Kawasaki and was determined to be in the range of 1.5-60.5 µg/ml for influenza B/Lee.

## Claims

1. A compound of the formula: 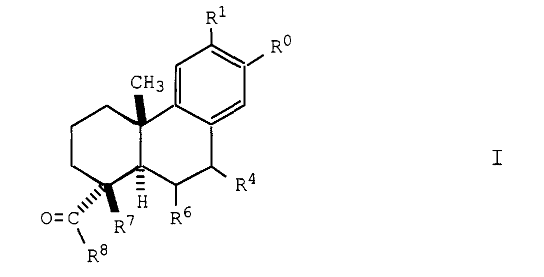 wherein:
R⁰ and R¹ are independently hydrogen, hydroxy, C₁-C₆ alkyl, C₁-C₆ alkoxy, hydroxy(C₁-C₆ alkyl), sulfhydryl, sulfamyl, -SO₂-Cl, -S-C(O)-N(CH₃)₂, amino, C₁-C₄ alkylamino, di(C₁-C₄ alkyl)amino, C₁-C₄ alkylsulfonylamino, di(C₁-C₄ alkylsulfonyl)amino -X⁰-O-C(O)-C₁-C₄ alkyl, -O-(X¹)ᵢ-X², -C(O)-X³, -N-C(O)-R² or -O-R³;
X⁰ is a bond or divalent(C₁-C₆ alkyl);
X¹ is an amino acid;
X² is hydrogen or an amino protecting group;
i is 1, 2 or 3;
X³ is C₁-C₆ alkyl, C₁-C₆ alkoxy, halo(C₁-C₆ alkyl), hydroxy(C₁-C₆ alkyl) or phenyl;
R² is C₁-C₄ alkyl, C₁-C₄ alkoxy, halo(C₁-C₄ alkyl), hydroxy(C₁-C₄ alkyl), phenyl, p-methoxy-phenyl, p-fluorophenyl, naphthyl, pyridyl, piperidinyl, thiazolyl, oxazolyl, thienyl, furyl, tetrahydrofuryl or cyclohexyl;
R³ is C₂-C₆ alkenyl, -CH₂-R^{3a}, -C(O)-R^{3b}, -C(S)-R^{3c}, -C(CH₃)₂C(O)NH₂, phenyl or a group of the formula: 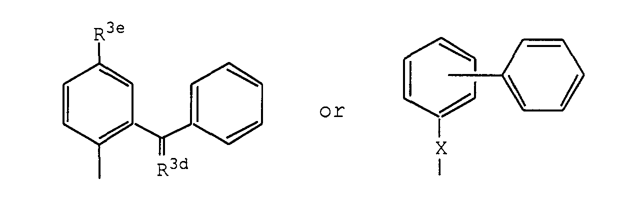
R^{3a} is phenyl, p-fluorophenyl, pyridyl, pyrrolidinyl, piperidinyl, piperazinyl, morpholinyl, N-(C₁-C₄ alkoxycarbonyl)piperidinyl, N-(trifluoromethyl)-piperidinyl, thiazolyl, oxazolyl, imidazolyl, isothiazolyl, isooxazolyl, quinolyl, isoquinolyl, thienyl, furyl, tetrahydrothienyl, tetrahydrofuryl, cyclohexyl, cyclopentyl, cyclopropyl or naphthyl;
R^{3b} is pyrrolidinyl, piperidinyl, piperazinyl, morpholinyl, N-(C₁-C₄ alkoxycarbonyl)piperidinyl, N-(trifluoromethyl)piperidinyl, benzyloxy, pyridylmethyloxy, C₁-C₆ alkoxy, halo(C₁-C₄ alkoxy), amino, C₁-C₄ alkylamino or di(C₁-C₄ alkyl)amino;
R^{3c} is amino, C₁-C₄ alkylamino or di(C₁-C₄ alkyl)amino;
R^{3d} is oxygen, hydroximino, hydrazino or =CHZ;
Z is hydrogen, C₁-C₄ alkyl, halogen, di(C₁-C₄ alkyl)amino, C₁-C₄ alkoxycarbonyl, carbamoyl(C₁-C₄ alkyl), N-(C₁-C₄ alkyl)carbamoyl or N,N-di(C₁-C₄ alkyl)carbamoyl;
R^{3e} is hydrogen, nitro or trifluoromethyl;
X is a bond or -(CH₂)-;
R⁴ is hydrogen, hydroxy, amino, C₁-C₄ alkylamino, di(C₁-C₄ alkyl)amino, C₁-C₄ alkoxy, =O, -O-S(CH₃)₂C(CH₃)₃, C₂-C₆ alkanoyloxy, N-(C₂-C₆ alkanoyl)amino, =N-R⁵ or R⁴ and R⁶ combine to form a bond;
R⁵ is hydroxy, amino, C₁-C₄ alkylamino, di(C₁-C₄ alkyl)amino, C₁-C₄ alkoxy, pyridylmethoxy, benzyloxy, piperazinyl, N-(methyl)piperazinyl or -O-CH₂-C(O)-R^{5a};
R^{5a} is hydroxy or C₁-C₄ alkoxy;
R⁶ is hydrogen, halo, C₁-C₄ alkyl or =O;
R⁷ is hydrogen or C₁-C₄ alkyl;
R⁸ is hydroxy, halo, C₁-C₆ alkoxy, pyrrolidinyl, piperidinyl, piperazinyl, 4-methyl-piperazinyl, morpholinyl or -N(R⁹)-R¹⁰;
R⁹ is hydrogen or methyl;
R¹⁰ is -(divalent C₁-C₆ alkyl)-R^{10a};
R^{10a} is pyridyl,
with the proviso that
i) when R⁴ is hydrogen or =O; R⁸ is methoxy; and R¹ is hydrogen; then R⁰ cannot be hydrogen, hydroxy, methoxy, -C(CH₃)₂OH, -C(O)CH₃, -OC(O)CH₃ or -C(CH₃)₂OC(O)CH₃;
ii) when R⁴ is hydrogen or =O; R⁸ is methoxy; and R⁰ is isopropyl; then R¹ cannot be hydroxy or methoxy;
iii) when R⁴ is hydrogen or =O; R⁸ is hydroxy; and R⁰ is hydrogen; then R¹ cannot be hydrogen or isopropyl;
iv) when R⁴ is hydrogen; R⁸ is methoxy; and R⁰ is hydrogen; then R¹ cannot be hydrogen, hydroxy, -C(O)CH₃ or -OC(O)CH₃;
v) when R¹ is hydrogen; and R⁰ is isopropyl; then R⁴ cannot be hydrogen, hydroxy, methoxy, ethoxy, =O, -OC(O)CH₃, =NOH or combine with R⁶ to form a bond;
vi) when R¹ is hydrogen; R⁴ is hydrogen and R⁸ is hydroxy; then R⁰ cannot be -C(CH₃)₂OC(O)CH₃;
or a pharmaceutically acceptable salt thereof.

2. A compound according to claim 1 where:
R⁰ is hydrogen, hydroxy, C₁-C₆ alkyl, C₁-C₆ alkoxy, hydroxy(C₁-C₆ alkyl), -X⁰-O-C(O)-C₁-C₄ alkyl, -O-(X¹)ᵢ-X², -C(O)-X³ or -O-R³;
R¹ is hydrogen, hydroxy, C₁-C₆ alkoxy, sulfhydryl, sulfamyl, -SO₂-Cl, amino, di(C₁-C₄ alkylsulfonyl)amino, -C(O)-X³, -N-C(O)-R² or -O-R³;
X⁰ is a bond or divalent(C₁-C₆ alkyl);
X¹ is an amino acid;
X² is hydrogen or an amino protecting group;
i is 1 or 2;
X³ is C₁-C₆ alkyl;
R² is hydroxy(C₁-C₄ alkyl);
R³ is C₂-C₆ alkenyl, -CH₂-R^{3a}, -C(O)-R^{3b}, -C(S)-R^{3c}, -C(CH₃)₂C(O)NH₂ or a group of the formula: 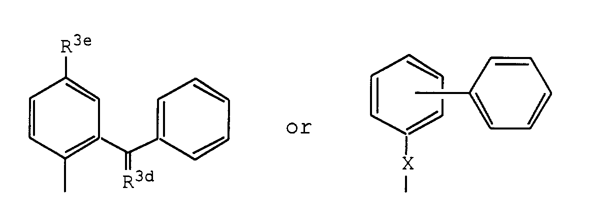
R^{3a} is phenyl, p-fluorophenyl, pyridyl, piperidinyl, piperazinyl or morpholinyl;
R^{3b} is piperidinyl, piperazinyl, morpholinyl, N-(C₁-C₄ alkoxycarbonyl)piperidinyl, N-(trifluoromethyl)piperidinyl, halo(C₁-C₄ alkoxy) or di(C₁-C₄ alkyl)amino;
R^{3c} is di(C₁-C₄ alkyl)amino;
R^{3d} is oxygen or hydroximino;
R^{3e} is hydrogen, nitro or trifluoromethyl;
X is a bond;
R⁴ is hydrogen, hydroxy, amino, =O, C₂-C₆ alkanoyloxy, =N-R⁵ or -OSi(CH₃)₂;
R⁵ is hydroxy, amino, di(C₁-C₄ alkyl)amino, C₁-C₄ alkoxy, pyridylmethoxy, N-(methyl)piperazinyl or -O-CH₂-C(O)-R^{5a};
R⁶ is hydrogen, chloro, bromo, methyl or =O;
R⁷ is hydrogen or methyl;
R⁸ is hydroxy, chloro, methoxy, 4-methylpiperazinyl or -N(R⁹)-R¹⁰;
R⁹ is hydrogen;
R¹⁰ is -CH₂-R^{10a}; and
R^{10a} is pyridyl;
or a pharmaceutically acceptable salt thereof.

3. A compound according to claim 2 where:
R⁰ is hydrogen, hydroxy, C₁-C₆ alkoxy, -O-(X¹)ᵢ-X², -X⁰-O-C(O)-C₁-C₄ alkyl or -O-R³;
R¹ is hydrogen, hydroxy, C₁-C₆ alkoxy or -O-R³;
X⁰ is a bond;
X¹ is an amino acid;
X² is hydrogen or an amino protecting group;
i is 1 or 2;
R³ is C₂-C₆ alkenyl, -CH₂-R^{3a} or -C(O)-R^{3b};
R^{3a} is p-fluorophenyl or pyridyl;
R^{3b} is piperidinyl;
R⁴ is hydrogen, hydroxy, =O or =N-R⁵;
R⁵ is hydroxy, dimethylamino or N-(methyl)piperazinyl;
R⁶ is hydrogen, bromo or =O;
R⁷ is methyl; and
R⁸ is methoxy;
or a pharmaceutically acceptable salt thereof.

4. A compound according to claim 3 where:
R⁰ is hydrogen, hydroxy, C₁-C₄ alkoxy, -O-(X¹)ᵢ-X², -O-C(O)methyl or -O-R³;
R¹ is hydrogen, hydroxy, C₁-C₄ alkoxy or -O-R³;
X¹ is glycine, alanine or valine;
X² is hydrogen, t-butoxycarbonyl or benzyloxycarbonyl;
R⁴ is =O or =N-R⁵;
R⁵ is hydroxy;
R⁶ is hydrogen;
or a pharmaceutically acceptable salt thereof.

## Patentansprüche

1. Verbindung der Formel 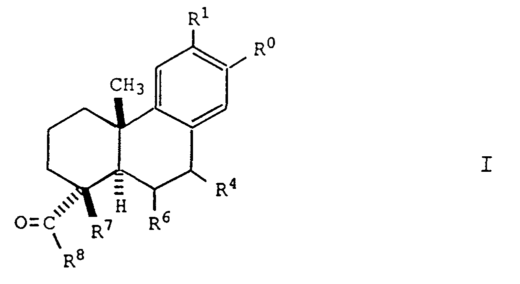 worin
R⁰ und R¹ unabhängig stehen für Wasserstoff, Hydroxy, C₁-C₆ Alkyl, C₁-C₆ Alkoxy, Hydroxy(C₁-C₆ Alkyl), Sulfhydryl, Sulfamyl, -SO₂-Cl, -S-C(O)-N(CH₃)₂, Amino, C₁-C₄ Alkylamino, Di(C₁-C₄ Alkyl)amino, C₁-C₄ Alkylsulfonylamino, Di(C₁-C₄ Alkylsulfonyl)amino, -X⁰-O-C(O)-C₁-C₄ Alkyl, -O-(X¹)ᵢ-X², -C(O)-X³, -N-C(O)-R² oder -O-R³,
X⁰ für eine Bindung oder divalentes C₁-C₆ Alkyl steht,
X¹ für eine Aminosäure steht,
X² für Wasserstoff oder eine Aminoschutzgruppe steht,
i für 1, 2 oder 3 steht,
X³ für C₁-C₆ Alkyl, C₁-C₆ Alkoxy, Halogen(C₁-C₆ Alkyl), Hydroxy(C₁-C₆ Alkyl) oder Phenyl steht,
R² steht für C₁-C₄ Alkyl, C₁-C₄ Alkoxy, Halogen(C₁-C₄ Alkyl), Hydroxy(C₁-C₄ Alkyl), Phenyl, p-Methoxyphenyl, p-Fluorphenyl, Naphthyl, Pyridyl, Piperidinyl, Thiazolyl, Oxazolyl, Thienyl, Furyl, Tetrahydrofuryl oder Cyclohexyl,
R³ für C₂-C₆ Alkenyl, -CH₂-R^{3a}, -C(O)-R^{3b}, -C(S)-R^{3c}, -C(CH₃)₂C(O)NH₂, Phenyl oder eine Gruppe der Formel steht: 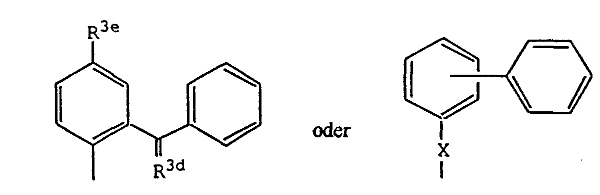
R^{3a} steht für Phenyl, p-Fluorphenyl, Pyridyl, Pyrrolidinyl, Piperidinyl, Piperazinyl, Morpholinyl, N-(C₁-C₄ Alkoxycarbonyl)piperidinyl, N-(Trifluormethyl)piperidinyl, Thiazolyl, Oxazolyl, Imidazolyl, Isothiazolyl, Isooxazolyl, Chinolyl, Isochinolyl, Thienyl, Furyl, Tetrahydrothienyl, Tetrahydrofuryl, Cyclohexyl, Cyclopentyl, Cyclopropyl oder Naphthyl,
R^{3b} steht für Pyrrolidinyl, Piperidinyl, Piperazinyl, Morpholinyl, N-(C₁-C₄ Alkoxycarbonyl)piperidinyl, N-(Trifluormethyl)piperidinyl, Benzyloxy, Pyridylmethyloxy, C₁-C₆ Alkoxy, Halogen(C₁-C₄ Alkoxy), Amino, C₁-C₄ Alkylamino oder Di(C₁-C₄ Alkyl)amino,
R^{3c} für Amino, C₁-C₄ Alkylamino oder Di(C₁-C₄ Alkyl)amino steht,
R^{3d} für Sauerstoff, Hydroximino, Hydrazino oder =CHZ steht,
Z für Wasserstoff, C₁-C₄ Alkyl, Halogen, Di(C₁-C₄ Alkyl)amino, C₁-C₄ Alkoxycarbonyl, Carbamoyl(C₁-C₄ Alkyl), N-(C₁-C₄ Alkyl)carbamoyl oder N,N-Di(C₁-C₄ Alkyl)carbamoyl steht,
R^{3e} für Wasserstoff, Nitro oder Trifluormethyl steht,
X für eine Bindung oder -(CH₂)- steht,
R⁴ für Wasserstoff, Hydroxy, Amino, C₁-C₄ Alkylamino, Di(C₁-C₄ Alkyl)amino, C₁-C₄ Alkoxy, =O, -O-S(CH₃)₂C(CH₃)₃, C₂-C₆ Alkanoyloxy, N-(C₂-C₆ Alkanoyl)amino oder =N-R⁵ steht, oder R⁴ und R⁶ unter Bildung einer Bindung kombineren,
R⁵ für Hydroxy, Amino, C₁-C₄ Alkylamino, Di(C₁-C₄ Alkyl)amino, C₁-C₄ Alkoxy, Pyridylmethoxy, Benzyloxy, Piperazinyl, N-(Methyl)piperazinyl oder -O-CH₂-C(O)-R^{5a} steht,
R^{5a} für Hydroxy oder C₁-C₄ Alkoxy steht,
R⁶ für Wasserstoff, Halogen, C₁-C₄ Alkyl oder =O steht,
R⁷ für Wasserstoff oder C₁-C₄ Alkyl steht,
R⁸ für Hydroxy, Halogen, C₁-C₆ Alkoxy, Pyrrolidinyl, Piperidinyl, Piperazinyl, 4-Methylpiperazinyl, Morpholinyl oder -N(R⁹)-R¹⁰ steht,
R⁹ für Wasserstoff oder Methyl steht,
R¹⁰ für -(divalentes C₁-C₆ Alkyl)-R^{10a} steht,
R^{10a} für Pyridyl steht,
mit der Maßgabe, daß
i) wenn R⁴ für Wasserstoff oder =O steht, R⁸ für Methoxy steht und R¹ für Wasserstoff steht, dann kann R⁰ nicht für Wasserstoff, Hydroxy, Methoxy, -C(CH₃)₂OH, -C(O)CH₃, -OC(O)CH₃ oder -C(CH₃)₂OC(O)CH₃ stehen,
ii) wenn R⁴ für Wasserstoff oder =O steht, R⁸ für Methoxy steht und R⁰ für Isopropyl steht, dann kann R¹ nicht für Hydroxy oder Methoxy stehen,
iii) wenn R⁴ für Wasserstoff oder =O steht, R⁸ für Hydroxy steht und R⁰ für Wasserstoff steht, dann kann R¹ nicht für Wasserstoff oder Isopropyl stehen,
iv) wenn R⁴ für Wasserstoff steht, R⁸ für Methoxy steht und R⁰ für Wasserstoff steht, dann kann R¹ nicht für Wasserstoff, Hydroxy, -C(O)CH₃ oder -OC(O)CH₃ stehen,
v) wenn R¹ für Wasserstoff steht und R⁰ für Isopropyl steht, dann kann R⁴ nicht für Wasserstoff, Hydroxy, Methoxy, Ethoxy, =O, -OC(O)CH₃ oder =NOH stehen oder mit R⁶ unter Bildung einer Bindung kombinieren,
vi) wenn R¹ für Wasserstoff steht, R⁴ für Wasserstoff steht und R⁸ für Hydroxy steht, dann kann R⁰ nicht für -C(CH₃)₂OC(O)CH₃ stehen,
oder ein pharmazeutisch annehmbares Salz hiervon.

2. Verbindung nach Anspruch 1, worin
R⁰ für Wasserstoff, Hydroxy, C₁-C₆ Alkyl, C₁-C₆ Alkoxy, Hydroxy(C₁-C₆ Alkyl), -X⁰-O-C(O)-C₁-C₄ Alkyl, -O-(X¹)ᵢ-X², -C(O)-X³ oder -O-R³ steht,
R¹ für Wasserstoff, Hydroxy, C₁-C₆ Alkoxy, Sulfhydryl, Sulfamyl, -SO₂-Cl, Amino, Di(C₁-C₄ Alkylsulfonyl)amino, -C(O)-X³, -N-C(O)-R² oder -O-R³ steht,
X⁰ für eine Bindung oder divalentes C₁-C₆ Alkyl steht,
X¹ für eine Aminosäure steht,
X² für Wasserstoff oder eine Aminoschutzgruppe steht,
i für 1 oder 2 steht,
X³ für C₁-C₆ Alkyl steht,
R² für Hydroxy(C₁-C₄ Alkyl) steht,
R³ für C₂-C₆ Alkenyl, -CH₂-R^{3a}, -C(O)-R^{3b}, -C(S)-R^{3c}, -C(CH₃)₂C(O)NH₂ oder eine Gruppe der Formel steht: 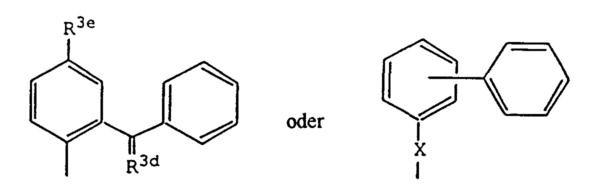
R^{3a} für Phenyl, p-Fluorphenyl, Pyridyl, Piperidinyl, Piperazinyl oder Morpholinyl steht,
R^{3b} für Piperidinyl, Piperazinyl, Morpholinyl, N-(C₁-C₄ Alkoxycarbonyl)piperidinyl, N-(Trifluormethyl)piperidinyl, Halogen(C₁-C₄ Alkoxy) oder Di(C₁-C₄ Alkyl)amino steht,
R^{3c} für Di(C₁-C₄ Alkyl)amino steht,
R^{3d} für Sauerstoff oder Hydroximino steht,
R^{3e} für Wasserstoff, Nitro oder Trifluormethyl steht,
X für eine Bindung steht,
R⁴ für Wasserstoff, Hydroxy, Amino, =O, C₂-C₆ Akanoyloxy, =N-R⁵ oder -OSi(CH₃)₂ steht,
R⁵ für Hydroxy, Amino, Di(C₁-C₄ Alkyl)amino, C₁-C₄ Alkoxy, Pyridylmethoxy, N-(Methyl)piperazinyl oder -O-CH₂-C(O)-R^{5a} steht,
R⁶ für Wasserstoff, Chor, Brom, Methyl oder =O steht,
R⁷ für Wasserstoff oder Methyl steht,
R⁸ für Hydroxy, Chlor, Methoxy, 4-Methylpiperazinyl oder -N(R⁹)-R¹⁰ steht,
R⁹ für Wasserstoff steht,
R¹⁰ für -CH₂-R^{10a} steht, und
R^{10a} für Pyridyl steht,
oder ein pharmazeutisch annehmbares Salz hiervon.

3. Verbindung nach Anspruch 2, worin
R⁰ für Wasserstoff, Hydroxy, C₁-C₆ Alkoxy, -O-(X¹)ᵢ-X², -X⁰-O-C(O)-C₁-C₄ Alkyl oder -O-R³ steht,
R¹ für Wasserstoff, Hydroxy, C₁-C₆ Alkoxy oder -O-R³ steht,
X⁰ für eine Bindung steht,
X¹ für eine Aminosäure steht,
X² für Wasserstoff oder eine Aminoschutzgruppe steht,
i für 1 oder 2 steht,
R³ für C₂-C₆ Alkenyl, -CH₂-R^{3a} oder -C(O)-R^{3b} steht,
R^{3a} für p-Fluorphenyl oder Pyridyl steht,
R^{3b} für Piperidinyl steht,
R⁴ für Wasserstoff, Hydroxy, =O oder =N-R⁵ steht,
R⁵ für Hydroxy, Dimethylamino oder N-(Methyl)piperazinyl steht,
R⁶ für Wasserstoff, Brom oder =O steht,
R⁷ für Methyl steht, und
R⁸ für Methoxy steht,
oder ein pharmazeutisch annehmbares Salz hiervon.

4. Verbindung nach Anspruch 3, worin
R⁰ für Wasserstoff, Hydroxy, C₁-C₄ Alkoxy, -O-(X¹)ᵢ-X², -O-C(O)-Methyl oder -O-R³ steht,
R¹ für Wasserstoff, Hydroxy, C₁-C₄ Alkoxy oder -O-R³ steht,
X¹ für Glycin, Alanin oder Valin steht,
X² für Wasserstoff, t-Butoxycarbonyl oder Benzyloxycarbonyl steht,
R⁴ für =O oder =N-R⁵ steht,
R⁵ für Hydroxy steht, und
R⁶ für Wasserstoff steht,
oder ein pharmazeutisch annehmbares Salz hiervon.

## Revendications

1. Composé répondant à la formule : 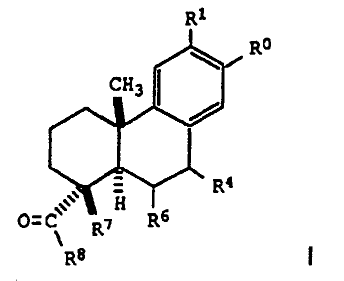 dans laquelle :
R⁰ et R¹ représentent indépendamment un atome d'hydrogène, un groupe hydroxyle, un groupe alkyle en C₁-C₆, un groupe alcoxy en C₁-C₆, un groupe hydroxy(alkyle en C₁-C₆), un groupe sulfhydryle, un groupe sulfamyle, un groupe -SO₂-Cl, un groupe -S-C(O)-N(CH₃)₂, un groupe amino, un groupe (alkyle en C₁-C₄)amino, un groupe di(alkyle en C₁-C₄)amino, un groupe (alkyle en C₁-C₄)sulfonylamino, un groupe di((alkyle en C₁-C₄)sulfonyl)amino, un groupe -X⁰-O-C(O)-(alkyle en C₁-C₄), un groupe -O-(X¹)ᵢ-X², un groupe -C(O)-X³, un groupe -N-C(O)-R² ou un groupe -O-R³ ;
X⁰ représente une liaison ou un groupe alkyle en C₁-C₆ divalent ;
X¹ représente un acide aminé ;
X² représente un atome d'hydrogène ou un groupe amino-protecteur ;
i vaut 1, 2 ou 3 ;
X³ représente un groupe alkyle en C₁-C₆, un groupe alcoxy en C₁-C₆, un groupe halogéno(alkyle en C₁-C₆), un groupe hydroxy(alkyle en C₁-C₆) ou un groupe phényle ;
R² représente un groupe alkyle en C₁-C₄, un groupe alcoxy en C₁-C₄, un groupe halogéno(alkyle en C₁-C₄), un groupe hydroxy(alkyle en C₁-C₄), un groupe phényle, un groupe p-méthoxyphényle, un groupe p-fluorophényle, un groupe naphtyle, un groupe pyridyle, un groupe pipéridinyle, un groupe thiazolyle, un groupe oxazolyle, un groupe thiényle, un groupe furyle, un groupe tétrahydrofuryle ou un groupe cyclohexyle ;
R³ représente un groupe alcényle en C₂-C₆, un groupe -CH₂-R^{3a}, un groupe -C(O)-R^{3b}, un groupe -C(S)-R^{3c}, un groupe -C(CH₃)₂C(O)NH₂, un groupe phényle ou un groupe répondant à la formule : 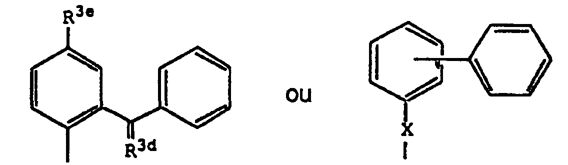
R^{3a} représente un groupe phényle, un groupe p-fluorophényle, un groupe pyridyle, un groupe pyrrolidinyle, un groupe pipéridinyle, un groupe pipérazinyle, un groupe morpholinyle, un groupe N-((alcoxy en C₁-C₄)-carbonyl)pipéridinyle, un groupe N-(trifluorométhyl)pipéridinyle, un groupe thiazolyle, un groupe oxazolyle, un groupe imidazolyle, un groupe isothiazolyle, un groupe isoxazolyle, un groupe quinolyle, un groupe isoquinolyle, un groupe thiényle, un groupe furyle, un groupe tétrahydrothiényle, un groupe tétrahydrofuryle, un groupe cyclohexyle, un groupe cyclopentyle, un groupe cyclopropyle ou un groupe naphtyle ;
R^{3b} représente un groupe pyrrolidinyle, un groupe pipéridinyle, un groupe pipérazinyle, un groupe morpholinyle, un groupe N-((alcoxy en C₁-C₄)-carbonyl)pipéridinyle, un groupe N-(trifluorométhyl)pipéridinyle, un groupe benzyloxy, un groupe pyridylméthyloxy, un groupe alcoxy en C₁-C₆, un groupe halogéno(alcoxy en C₁-C₄), un groupe amino, un groupe (alkyle en C₁-C₄)amino ou un groupe di(alkyle en C₁-C₄)amino ;
R^{3c} représente un groupe amino, un groupe (alkyle en C₁-C₄)amino ou un groupe di(alkyle en C₁-C₄)amino ;
R^{3d} représente un atome d'oxygène, un groupe hydroximino, un groupe hydrazino ou un groupe =CHZ ;
Z représente un atome d'hydrogène, un groupe alkyle en C₁-C₄, un atome d'halogène, un groupe di(alkyle en C₁-C₄)amino, un groupe (alcoxy en C₁-C₄)carbonyle, un groupe carbamoyl(alkyle en C₁-C₄), un groupe N-(alkyle en C₁-C₄)carbamoyle ou un groupe N,N-di(alkyle en C₁-C₄)carbamoyle ;
R^{3e} représente un atome d'hydrogène, un groupe nitro ou un groupe trifluorométhyle ;
X représente une liaison ou un groupe -(CH₂)- ;
R⁴ représente un atome d'hydrogène, un groupe hydroxyle, un groupe amino, un groupe (alkyle en C₁-C₄)amino, un groupe di(alkyle en C₁-C₄)amino, un groupe alcoxy en C₁-C₄, un groupe =O, un groupe -O-S(CH₃)₂C(CH₃)₃, un groupe (alcanoyle en C₂-C₆)oxy, un groupe N-(alcanoyle en C₂-C₆)amino, un groupe =N-R⁵ ou R⁴ et R⁶ se combinent pour former une liaison ;
R⁵ représente un groupe hydroxyle, un groupe amino, un groupe (alkyle en C₁-C₄)amino, un groupe di(alkyle en C₁-C₄)amino, un groupe alcoxy en C₁-C₄, un groupe pyridylméthoxy, un groupe benzyloxy, un groupe pipérazinyle, un groupe N-(méthyl)pipérazinyle ou un groupe -O-CH₂-C(O)-R^{5a} ;
R^{5a} représente un groupe hydroxyle ou un groupe alcoxy en C₁-C₄;
R⁶ représente un atome d'hydrogène, un atome d'halogène, un groupe alkyle en C₁-C₄ ou un groupe =O ;
R⁷ représente un atome d'hydrogène ou un groupe alkyle en C₁-C₄ ;
R⁸ représente un groupe hydroxyle, un atome d'halogène, un groupe alcoxy en C₁-C₆, un groupe pyrrolidinyle, un groupe pipéridinyle, un groupe pipérazinyle, un groupe 4-méthylpipérazinyle, un groupe morpholinyle ou un groupe -N(R⁹)-R¹⁰ ;
R⁹ représente un atome d'hydrogène ou un groupe méthyle ;
R¹⁰ représente un groupe -(alkyle en C₁-C₆ divalent)-R^{10a} ;
R^{10a} représente un groupe pyridyle,
pour autant que
i) lorsque R⁴ représente un atome d'hydrogène ou un groupe =O ; R⁸ représente un groupe méthoxy ; et R¹ représente un atome d'hydrogène ; alors R⁰ ne peut pas représenter un atome d'hydrogène, un groupe hydroxyle, un groupe méthoxy, un groupe -C(CH₃)₂OH, un groupe -C(O)CH₃, un groupe -OC(O)CH₃ ou un groupe -C(CH₃)₂OC(O)CH₃ ;
ii) lorsque R⁴ représente un atome d'hydrogène ou un groupe =O ; R⁸ représente un groupe méthoxy ; et R⁰ représente un groupe isopropyle ; alors R¹ ne peut pas représenter un groupe hydroxyle ou un groupe méthoxy ;
iii) lorsque R⁴ représente un atome d'hydrogène ou un groupe =O ; R⁸ représente un groupe hydroxyle ; et R⁰ représente un atome d'hydrogène ; alors R¹ ne peut pas représenter un atome d'hydrogène ou un groupe isopropyle;
iv) lorsque R⁴ représente un atome d'hydrogène ; R⁸ représente un groupe méthoxy ; et R⁰ représente un atome d'hydrogène ; alors R¹ ne peut pas représenter un atome d'hydrogène, un groupe hydroxyle, un groupe -C(O)CH₃ ou un groupe -OC(O)CH₃ ;
v) lorsque R¹ représente un atome d'hydrogène ; et R⁰ représente un groupe isopropyle ; alors R⁴ ne peut pas représenter un atome d'hydrogène, un groupe hydroxyle, un groupe méthoxy, un groupe éthoxy, un groupe =O, un groupe -OC(O)CH₃, un groupe =NOH ou se combiner avec R⁶ pour former une liaison ;
vi) lorsque R¹ représente un atome d'hydrogène ; R⁴ représente un atome d'hydrogène et R⁸ représente un groupe hydroxyle ; alors R⁰ ne peut pas représenter un groupe -C(CH₃)₂OC(O)CH₃ ;
ou un sel pharmaceutiquement acceptable de celui-ci.

2. Composé selon la revendication 1, dans lequel :
R⁰ représente un atome d'hydrogène, un groupe hydroxyle, un groupe alkyle en C₁-C₆, un groupe alcoxy en C₁-C₆, un groupe hydroxy(alkyle en C₁-C₆), un groupe -X⁰-O-C(O)-(alkyle en C₁-C₄), un groupe -O-(X¹)ᵢ-X², un groupe -C(O)-X³ ou un groupe -O-R³ ;
R¹ représente un atome d'hydrogène, un groupe hydroxyle, un groupe alcoxy en C₁-C₆, un groupe sulfhydryle, un groupe sulfamyle, un groupe -SO₂-Cl, un groupe amino, un groupe di((alkyle en C₁-C₄)sulfonyl)amino, un groupe -C(O)-X³, un groupe -N-C(O)-R² ou un groupe -O-R³ ;
X⁰ représente une liaison ou un groupe alkyle en C₁-C₆ divalent ;
X¹ représente un acide aminé ;
X² représente un atome d'hydrogène ou un groupe amino-protecteur ;
i vaut 1 ou 2 ;
X³ représente un groupe alkyle en C₁-C₆;
R² représente un groupe hydroxy(alkyle en C₁-C₄) ;
R³ représente un groupe alcényle en C₂-C₆, un groupe -CH₂-R^{3a}, un groupe -C(O)-R^{3b}, un groupe -C(S)-R^{3c}, un groupe -C(CH₃)₂C(O)NH₂ ou un groupe répondant à la formule: 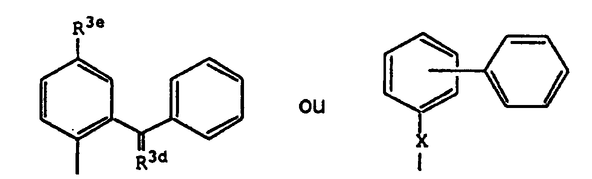
R^{3a} représente un groupe phényle, un groupe p-fluorophényle, un groupe pyridyle, un groupe pipéridinyle, un groupe pipérazinyle ou un groupe morpholinyle ;
R^{3b} représente un groupe pipéridinyle, un groupe pipérazinyle, un groupe morpholinyle, un groupe N-((alcoxy en C₁-C₄)carbonyl)pipéridinyle, un groupe N-(trifluorométhyl)pipéridinyle, un groupe halogéno(alcoxy en C₁-C₄) ou un groupe di(alkyle en C₁-C₄)amino ;
R^{3c} représente un groupe di(alkyle en C₁-C₄)amino ;
R^{3d} représente un atome d'oxygène ou un groupe hydroximino ;
R^{3e} représente un atome d'hydrogène, un groupe nitro ou un groupe trifluorométhyle ;
X représente une liaison ;
R⁴ représente un atome d'hydrogène, un groupe hydroxyle, un groupe amino, un groupe =O, un groupe (alcanoyle en C₂-C₆)oxy, un groupe =N-R⁵ ou un groupe -OSi(CH₃)₂ ;
R⁵ représente un groupe hydroxyle, un groupe amino, un groupe di(alkyle en C₁-C₄)amino, un groupe alcoxy en C₁-C₄, un groupe pyridylméthoxy, un groupe N-(méthyl)pipérazinyle ou un groupe -O-CH₂-C(O)-R^{5a} ;
R⁶ représente un atome d'hydrogène, un atome de chlore, un atome de brome, un groupe méthyle ou un groupe =O ;
R⁷ représente un atome d'hydrogène ou un groupe méthyle ;
R⁸ représente un groupe hydroxyle, un atome de chlore, un groupe méthoxy, un groupe 4-méthylpipérazinyle ou un groupe -N(R⁹)-R¹⁰ ;
R⁹ représente un atome d'hydrogène ;
R¹⁰ représente un groupe -CH₂-R^{10a} ; et
R^{10a} représente un groupe pyridyle ;
ou un sel pharmaceutiquement acceptable de celui-ci.

3. Composé selon la revendication 2, dans lequel :
R⁰ représente un atome d'hydrogène, un groupe hydroxyle, un groupe alcoxy en C₁-C₆, un groupe -O-(X¹)ᵢ-X², un groupe -X⁰-O-C(O)-(alkyle en C₁-C₄) ou un groupe -O-R³ ;
R¹ représente un atome d'hydrogène, un groupe hydroxyle, un groupe alcoxy en C₁-C₆ ou un groupe -O-R³ ;
X⁰ représente une liaison ;
X¹ représente un acide aminé ;
X² représente un atome d'hydrogène ou un groupe amino-protecteur ;
i vaut 1 ou 2 ;
R³ représente un groupe alcényle en C₂-C₆, un groupe -CH₂-R^{3a} ou un groupe -C(O)-R^{3b} ;
R^{3a} représente un groupe p-fluorophényle ou un groupe pyridyle ;
R^{3b} représente un groupe pipéridinyle ;
R⁴ représente un atome d'hydrogène, un groupe hydroxyle, un groupe =O ou un groupe =N-R⁵ ;
R⁵ représente un groupe hydroxyle, un groupe diméthylamino ou un groupe N-(méthyl)pipérazinyle ;
R⁶ représente un atome d'hydrogène, un atome de brome ou un groupe =O ;
R⁷ représente un groupe méthyle ; et
R⁸ représente un groupe méthoxy ;
ou un sel pharmaceutiquement acceptable de celui-ci.

4. Composé selon la revendication 3, dans lequel :
R⁰ représente un atome d'hydrogène, un groupe hydroxyle, un groupe alcoxy en C₁-C₄, un groupe -O-(X¹)ᵢ-X², un groupe -O-C(O)méthyle ou un groupe -O-R³ ;
R¹ représente un atome d'hydrogène, un groupe hydroxyle, un groupe alcoxy en C₁-C₄ ou un groupe -O-R³ ;
X¹ représente la glycine, l'alanine ou la valine ;
X² représente un atome d'hydrogène, un groupe t-butoxycarbonyle ou un groupe benzyloxycarbonyle ;
R⁴ représente un groupe =O ou un groupe =N-R⁵ ;
R⁵ représente un groupe hydroxyle ;
R⁶ représente un atome d'hydrogène ;
ou un sel pharmaceutiquement acceptable de celui-ci.
